# EUROPEAN PATENT APPLICATION

(11) **EP 3 868 777 A1**
(43) Date of publication of application: **25.08.2021**
(21) Application number: 21163171.8
(22) Date of filing: 24.06.2016
(51) Int. Cl.: C07K 14/415, G01N 33/53, A61K 39/35, A61P 37/08

(54) **IMMUNOGENIC COMPOSITION**

(30) Priority: 26.06.2015 EP 15306013
(62) Divisional of application: 16733421.8
(71) Applicant: Institut national de recherche pour l'agriculture, l'alimentation et l'environnement, 75007 Paris (FR)
(72) Inventor: DENERY, Sandra, 44300 NANTES (FR); MAMERI, Hamza, 93500 PANTIN (FR); GAUDIN, Jean-Charles, 45000 ORLEANS (FR); SNEGAROFF, Jacques, 78180 MONTIGNY-LE-BRETONNEUX (FR); BROSSARD, Chantal, 44240 LA CHAPELLE SUR ERDRE (FR); LARRE, Colette, 44300 NANTES (FR); TRANQUET, Olivier, 44240 LA CHAPELLE SUR ERDRE (FR)
(74) Representative: Nony

(57) **Abstract**

The invention concerns an immunogenic composition suitable for desensitization of an individual with diagnosed IgE-dependent gluten allergy comprising:
- a polypeptide or a mixture of polypeptides comprising the N-terminal domain of an α-gliadin, or a fragment of the N-terminal domain of an α-gliadin , wherein the polypeptide has at least 70% amino acid identity with the polypeptide of SEQ ID NO: 5 or with a polypeptide having a length ranging from 25 to 200 amino acid residues of SEQ ID NO: 5; and
- at least one physiologically acceptable adjuvant.

SEQUENCE LISTING

<110> INSTITUT NATIONAL DE LA RECHERCHE AGRONOMIQUE

<120> immunogenic composition

<130> PR75317

<150> EP15306013.2
<151> 2015-06-26

<160> 92

<170> BiSSAP 1.3.6

<210> 1
<211> 263
<212> PRT
<213> Triticum aestivum

<220>
<223> natural alpha-gliadin

<400> 1
   Val Arg Val Pro Val Pro Gln Leu Gln Pro Gln Asn Pro Ser Gln Gln
   1               5                   10                  15
   Gln Pro Gln Glu Gln Val Pro Leu Val Gln Gln Gln Gln Phe Leu Gly
               20                  25                  30
   Gln Gln Gln Pro Phe Pro Pro Gln Gln Pro Tyr Pro Gln Pro Gln Pro
           35                  40                  45
   Phe Pro Ser Gln Gln Pro Tyr Leu Gln Leu Gln Pro Phe Pro Gln Pro
       50                  55                  60
   Gln Leu Pro Tyr Pro Gln Pro Gln Pro Phe Arg Pro Gln Gln Pro Tyr
   65                  70                  75                  80
   Pro Gln Pro Gln Pro Gln Tyr Ser Gln Pro Gln Gln Pro Ile Ser Gln
                   85                  90                  95
   Gln Gln Gln Gln Gln Gln Gln Gln Gln Gln Gln Gln Gln Gln Ile Leu
               100                 105                 110
   Gln Gln Ile Leu Gln Gln Gln Leu Ile Pro Cys Met Asp Val Val Leu
           115                 120                 125
   Gln Gln His Asn Ile Val His Gly Arg Ser Gln Val Leu Gln Gln Ser
       130                 135                 140
   Thr Tyr Gln Leu Leu Arg Glu Leu Cys Cys Gln His Leu Trp Gln Ile
   145                 150                 155                 160
   Pro Glu Gln Ser Gln Cys Gln Ala Ile His Asn Val Val His Ala Ile
                   165                 170                 175
   Ile Leu His Gln Gln Gln Lys Gln Gln Gln Gln Pro Ser Ser Gln Val
               180                 185                 190
   Ser Phe Gln Gln Pro Leu Gln Gln Tyr Pro Leu Gly Gln Gly Ser Phe
           195                 200                 205
   Arg Pro Ser Gln Gln Asn Pro Gln Ala Gln Gly Ser Val Gln Pro Gln
       210                 215                 220
   Gln Leu Pro Gln Phe Glu Glu Ile Arg Asn Leu Ala Leu Gln Thr Leu
   225                 230                 235                 240
   Pro Ala Met Cys Asn Val Tyr Ile Pro Pro Tyr Cys Thr Ile Ala Pro
                   245                 250                 255
   Phe Gly Ile Phe Gly Thr Asn
               260

<210> 2
<211> 308
<212> PRT

<213> Triticum aestivum

<220>
<223> natural gamma-gliadin

<400> 2
Asn Ile Gln Val Asp Pro Ser Gly Gln Val Gln Trp Leu Gln Gln Gln
1               5                   10                  15
Leu Val Pro Gln Leu Gln Gln Pro Leu Ser Gln Gln Pro Gln Gln Thr
            20                  25                  30
Phe Pro Gln Pro Gln Gln Thr Phe Pro His Gln Pro Gln Gln Gln Val
        35                  40                  45
Pro Gln Pro Gln Gln Pro Gln Gln Pro Phe Leu Gln Pro Gln Gln Pro
    50                  55                  60
Phe Pro Gln Gln Pro Gln Gln Pro Phe Pro Gln Thr Gln Gln Pro Gln
65                  70                  75                  80
Gln Pro Phe Pro Gln Gln Pro Gln Gln Pro Phe Pro Gln Thr Gln Gln
                85                  90                  95
Pro Gln Gln Pro Phe Pro Gln Gln Pro Gln Gln Pro Phe Pro Gln Thr
            100                 105                 110
Gln Gln Pro Gln Gln Pro Phe Pro Gln Leu Gln Gln Pro Gln Gln Pro
        115                 120                 125
Phe Pro Gln Pro Gln Gln Gln Leu Pro Gln Pro Gln Gln Pro Gln Gln
    130                 135                 140
Ser Phe Pro Gln Gln Gln Arg Pro Phe Ile Gln Pro Ser Leu Gln Gln
145                 150                 155                 160
Gln Leu Asn Pro Cys Lys Asn Ile Leu Leu Gln Gln Cys Lys Pro Ala
                165                 170                 175
Ser Leu Val Ser Ser Leu Trp Ser Ile Ile Trp Pro Gln Ser Asp Cys
            180                 185                 190
Gln Val Met Arg Gln Gln Cys Cys Gln Gln Leu Ala Gln Ile Pro Gln
        195                 200                 205
Gln Leu Gln Cys Ala Ala Ile His Ser Val Val His Ser Ile Ile Met
    210                 215                 220
Gln Gln Gln Gln Gln Gln Gln Gln Gln Gln Gly Ile Asp Ile Phe Leu
225                 230                 235                 240
Pro Leu Ser Gln His Glu Gln Val Gly Gln Gly Ser Leu Val Gln Gly
                245                 250                 255
Gln Gly Ile Ile Gln Pro Gln Gln Pro Ala Gln Leu Glu Ala Ile Arg
            260                 265                 270
Ser Leu Val Leu Gln Thr Leu Pro Ser Met Cys Asn Val Tyr Val Pro
        275                 280                 285
Pro Glu Cys Ser Ile Met Arg Ala Pro Phe Ala Ser Ile Val Ala Gly
    290                 295                 300
Ile Gly Gly Gln
305

<210> 3
<211> 283
<212> PRT
<213> Artificial Sequence

<220>
<223> recombinant alpha-gliadin

<400> 3
Gly Ser Ser His His His His His His Ser Gln Asp Pro Val Arg Val
1               5                   10                  15
Pro Met Pro Gln Leu Gln Pro Gln Asn Pro Ser Gln Gln Gln Pro Gln
            20                  25                  30
Glu Gln Val Pro Leu Val Gln Gln Gln Gln Phe Pro Gly Gln Gln Gln

35                  40                  45
Pro Phe Pro Pro Gln Gln Pro Tyr Pro Gln Pro Gln Pro Phe Pro Ser
    50                  55                  60
Gln Gln Pro Tyr Leu Gln Leu Gln Pro Phe Pro Gln Pro Gln Leu Pro
65                  70                  75                  80
Tyr Pro Gln Pro Gln Pro Phe Arg Pro Gln Gln Pro Tyr Pro Gln Pro
                85                  90                  95
Gln Pro Gln Tyr Ser Gln Pro Gln Gln Pro Ile Ser Gln Gln Gln Gln
            100                 105                 110
Gln Gln Gln Gln Gln Gln Gln Gln Gln Gln Gln Ile Leu Gln Gln Ile
        115                 120                 125
Leu Gln Gln Gln Leu Ile Pro Cys Arg Asp Val Val Leu Gln Gln
    130                 135                 140
His Asn Ile Ala His Ala Ser Ser Gln Val Leu Gln Gln Ser Ser Tyr
145                 150                 155                 160
Gln Leu Leu Gln Gln Leu Cys Cys Gln Arg Leu Trp Gln Ile Pro Glu
                165                 170                 175
Gln Ser Arg Cys Gln Ala Ile His Asn Val Val His Ala Ile Ile Leu
            180                 185                 190
Gln Gln Gln Gln Gln Gln Gln Gln Gln Gln Gln Gln Pro Ser Ser Gln
        195                 200                 205
Val Ser Tyr Gln Gln Pro Gln Gln Gln Tyr Pro Ser Gly Gln Gly Ser
    210                 215                 220
Phe Gln Pro Ser Gln Gln Asn Ser Gln Ala Gln Gly Ser Val Gln Ser
225                 230                 235                 240
Gln Gln Leu Pro Gln Phe Glu Glu Ile Arg Asn Leu Val Leu Gln Thr
                245                 250                 255
Leu Pro Ala Met Cys Asn Val Tyr Ile Pro Pro Tyr Cys Ser Thr Thr
            260                 265                 270
Ile Ala Pro Phe Gly Ile Phe Gly Thr Asn Ser
        275                 280

<210> 4
<211> 274
<212> PRT
<213> Artificial Sequence

<220>
<223> recombinant gamma-gliadin

<400> 4
Asn Met Gln Val Asp Pro Ser Gly Gln Val Gln Trp Pro Gln Gln Gln
1               5                   10                  15
Pro Phe Arg Gln Pro Gln Gln Pro Phe Tyr Gln Gln Pro Gln Gln Thr
            20                  25                  30
Phe Pro Gln Pro Gln Gln Thr Phe Pro His Gln Pro Gln Gln Gln Phe
        35                  40                  45
Pro Gln Pro Gln Gln Pro Gln Gln Gln Phe Pro Gln Pro Gln Gln Pro
    50                  55                  60
Gln Gln Pro Phe Pro Gln Pro Gln Gln Ala Gln Leu Pro Phe Pro Gln
65                  70                  75                  80
Gln Pro Gln Gln Pro Phe Pro Gln Pro Gln Gln Pro Gln Gln Pro Phe
                85                  90                  95
Pro Gln Ser Gln Gln Pro Gln Gln Pro Phe Pro Gln Pro Gln Gln Pro
            100                 105                 110
Gln Gln Ser Phe Pro Gln Gln Gln Gln Pro Leu Ile Gln Pro Tyr Leu
        115                 120                 125
Gln Gln Gln Met Asn Pro Cys Lys Asn Tyr Leu Leu Gln Gln Cys Asn
    130                 135                 140
Pro Val Ser Leu Val Ser Ser Leu Val Ser Met Ile Leu Pro Arg Ser
145                 150                 155                 160
Asp Cys Gln Val Met Gln Gln Gln Cys Cys Gln Gln Leu Ala Gln Ile

165                 170                 175
Pro Arg Gln Leu Gln Cys Ala Ala Ile His Ser Val Val His Ser Ile
            180                 185                 190
Ile Met Gln Gln Glu Gln Gln Gln Gly Ile Gln Ile Leu Arg Pro Leu
        195                 200                 205
Phe Gln Leu Val Gln Gly Gln Gly Ile Ile Gln Pro Gln Gln Pro Ala
    210                 215                 220
Gln Tyr Glu Val Ile Arg Ser Leu Val Leu Arg Thr Leu Pro Asn Met
225                 230                 235                 240
Cys Asn Val Tyr Val Arg Pro Asp Cys Ser Thr Ile Asn Ala Pro Phe
                245                 250                 255
Ala Ser Ile Val Ala Gly Ile Ser Gly Gln Leu Glu His His His His
            260                 265                 270
His His

<210> 5
<211> 144
<212> PRT
<213> Artificial Sequence

<220>
<223> recombinant N-terminal domain of alpha-gliadin

<400> 5
   Gly Ser Ser His His His His His His Ser Gln Asp Pro Val Arg Val
   1               5                   10                  15
   Pro Met Pro Gln Leu Gln Pro Gln Asn Pro Ser Gln Gln Gln Pro Gln
               20                  25                  30
   Glu Gln Val Pro Leu Val Gln Gln Gln Gln Phe Pro Gly Gln Gln Gln
           35                  40                  45
   Pro Phe Pro Pro Gln Gln Pro Tyr Pro Gln Pro Gln Pro Phe Pro Ser
       50                  55                  60
   Gln Gln Pro Tyr Leu Gln Leu Gln Pro Phe Pro Gln Pro Gln Leu Pro
   65                  70                  75                  80
   Tyr Pro Gln Pro Gln Pro Phe Arg Pro Gln Gln Pro Tyr Pro Gln Pro
                   85                  90                  95
   Gln Pro Gln Tyr Ser Gln Pro Gln Gln Pro Ile Ser Gln Gln Gln Gln
               100                 105                 110
   Gln Gln Gln Gln Gln Gln Gln Gln Gln Gln Gln Ile Leu Gln Gln Ile
           115                 120                 125
   Leu Gln Gln Gln Gln Leu Ile Pro Cys Arg Asp Val Val Leu Gln Gln
       130                 135                 140

<210> 6
<211> 134
<212> PRT
<213> Artificial Sequence

<220>
<223> recombinant N-terminal domain of gamma-gliadin

<400> 6
Asn Met Gln Val Asp Pro Ser Gly Gln Val Gln Trp Pro Gln Gln Gln
1               5                   10                  15
Pro Phe Arg Gln Pro Gln Gln Pro Phe Tyr Gln Gln Pro Gln Gln Thr
            20                  25                  30
Phe Pro Gln Pro Gln Gln Thr Phe Pro His Gln Pro Gln Gln Gln Phe
        35                  40                  45

Pro Gln Pro Gln Gln Pro Gln Gln Gln Phe Pro Gln Pro Gln Gln Pro
    50                  55                  60
Gln Gln Pro Phe Pro Gln Pro Gln Gln Ala Gln Leu Pro Phe Pro Gln
65                  70                  75                  80
Gln Pro Gln Gln Pro Phe Pro Gln Pro Gln Gln Pro Gln Gln Pro Phe
                85                  90                  95
Pro Gln Ser Gln Gln Pro Gln Gln Pro Phe Pro Gln Pro Gln Gln Pro
            100                 105                 110
Gln Gln Ser Phe Pro Gln Gln Gln Gln Pro Leu Ile Gln Pro Tyr Leu
        115                 120                 125
Gln Gln Gln Met Asn Pro
    130

<210> 7
<211> 151
<212> PRT
<213> Artificial Sequence

<220>
<223> recombinant C-terminal domain of alpha-gliadin

<400> 7
Gln Leu Ile Pro Cys Arg Asp Val Val Leu Gln Gln His Asn Ile Ala
1               5                   10                  15
His Ala Ser Ser Gln Val Leu Gln Gln Ser Ser Tyr Gln Leu Leu Gln
            20                  25                  30
Gln Leu Cys Cys Gln Arg Leu Trp Gln Ile Pro Glu Gln Ser Arg Cys
        35                  40                  45
Gln Ala Ile His Asn Val Val His Ala Ile Ile Leu Gln Gln Gln Gln
    50                  55                  60
Gln Gln Gln Gln Gln Gln Gln Gln Pro Ser Ser Gln Val Ser Tyr Gln
65                  70                  75                  80
Gln Pro Gln Gln Gln Tyr Pro Ser Gly Gln Gly Ser Phe Gln Pro Ser
                85                  90                  95
Gln Gln Asn Ser Gln Ala Gln Gly Ser Val Gln Ser Gln Gln Leu Pro
            100                 105                 110
Gln Phe Glu Glu Ile Arg Asn Leu Val Leu Gln Thr Leu Pro Ala Met
        115                 120                 125
Cys Asn Val Tyr Ile Pro Pro Tyr Cys Ser Thr Thr Ile Ala Pro Phe
    130                 135                 140
Gly Ile Phe Gly Thr Asn Ser
145                 150

<210> 8
<211> 144
<212> PRT
<213> Artificial Sequence

<220>
<223> recombinant C-terminal domain of gamma-gliadin

<400> 8
Gln Met Asn Pro Cys Lys Asn Tyr Leu Leu Gln Gln Cys Asn Pro Val
1               5                   10                  15
Ser Leu Val Ser Ser Leu Val Ser Met Ile Leu Pro Arg Ser Asp Cys
            20                  25                  30
Gln Val Met Gln Gln Gln Cys Cys Gln Gln Leu Ala Gln Ile Pro Arg
        35                  40                  45
Gln Leu Gln Cys Ala Ala Ile His Ser Val Val His Ser Ile Ile Met
    50                  55                  60
Gln Gln Glu Gln Gln Gln Gly Ile Gln Ile Leu Arg Pro Leu Phe Gln

65                  70                  75                  80
Leu Val Gln Gly Gln Gly Ile Ile Gln Pro Gln Gln Pro Ala Gln Tyr
                85                  90                  95
Glu Val Ile Arg Ser Leu Val Leu Arg Thr Leu Pro Asn Met Cys Asn
            100                 105                 110
Val Tyr Val Arg Pro Asp Cys Ser Thr Ile Asn Ala Pro Phe Ala Ser
        115                 120                 125
Ile Val Ala Gly Ile Ser Gly Gln Leu Glu His His His His His His
    130                 135                 140

<210> 9
<211> 10
<212> PRT
<213> Artificial Sequence

<220>
<223> N-terminal peptide of alpha-gliadin

<400> 9
Val Arg Val Pro Val Pro Gln Leu Gln Pro
1               5                   10

<210> 10
<211> 18
<212> PRT
<213> Artificial Sequence

<220>
<223> N-terminal peptide of alpha-gliadin

<400> 10
Val Gln Gln Gln Gln Phe Pro Gly Gln Gln Gln Gln Phe Pro Pro Gln
1               5                   10                  15
Gln Pro

<210> 11
<211> 20
<212> PRT
<213> Artificial Sequence

<220>
<223> N-terminal peptide of alpha-gliadin

<400> 11
Gln Pro Tyr Leu Gln Leu Gln Pro Phe Pro Gln Pro Gln Pro Phe Pro
1               5                   10                  15
Pro Gln Leu Pro
            20

<210> 12
<211> 10
<212> PRT
<213> Artificial Sequence

<220>
<223> N-terminal peptide of alpha-gliadin

<400> 12
Tyr Leu Gln Leu Gln Pro Phe Pro Gln Pro
1               5                   10

<210> 13
<211> 14
<212> PRT
<213> Artificial Sequence

<220>
<223> N-terminal peptide of alpha-gliadin

<400> 13
Pro Leu Val Gln Gln Gln Gln Phe Pro Gly Gln Gln Gln Gln
1               5                   10

<210> 14
<211> 10
<212> PRT
<213> Artificial Sequence

<220>
<223> N-terminal peptide of alpha-gliadin

<400> 14
Gln Gln Gln Phe Pro Gly Gln Gln Gln Gln
1               5                   10

<210> 15
<211> 38
<212> PRT
<213> Artificial Sequence

<220>
<223> N-terminal peptide of alpha-gliadin

<400> 15
Gln Pro Tyr Pro Gln Pro Gln Pro Phe Pro Ser Gln Gln Pro Tyr Leu
1               5                   10                  15
Gln Leu Gln Pro Phe Pro Gln Pro Gln Pro Phe Pro Pro Gln Leu Pro
            20                  25                  30
Tyr Pro Gln Pro Gln Ser
        35

<210> 16
<211> 36
<212> PRT
<213> Artificial Sequence

<220>
<223> N-terminal peptide of alpha-gliadin

<400> 16
Gln Pro Tyr Pro Gln Pro Gln Pro Phe Pro Ser Gln Gln Pro Tyr Leu
1               5                   10                  15
Gln Leu Gln Pro Phe Pro Gln Pro Gln Pro Phe Pro Pro Gln Leu Pro
            20                  25                  30
Tyr Pro Gln Pro

        35

<210> 17
<211> 10
<212> PRT
<213> Artificial Sequence

<220>
<223> N-terminal peptide of alpha-gliadin

<400> 17
Gln Pro Gln Pro Phe Pro Ser Gln Gln Pro
1               5                   10

<210> 18
<211> 30
<212> PRT
<213> Artificial Sequence

<220>
<223> N-terminal peptide of alpha-gliadin

<400> 18
Val Gln Gln Gln Gln Phe Pro Gly Gln Gln Gln Gln Phe Pro Pro Gln
1               5                   10                  15
Gln Pro Tyr Pro Gln Pro Gln Pro Phe Pro Ser Gln Gln Pro
            20                  25                  30

<210> 19
<211> 14
<212> PRT
<213> Artificial Sequence

<220>
<223> N-terminal peptide of alpha-gliadin

<400> 19
Gln Ser Phe Pro Pro Gln Gln Pro Tyr Pro Gln Gln Gln Pro
1               5                   10

<210> 20
<211> 12
<212> PRT
<213> Artificial Sequence

<220>
<223> N-terminal peptide of alpha-gliadin

<400> 20
Pro Gln Gln Pro Ile Ser Gln Gln Gln Ala Gln Gln
1               5                   10

<210> 21
<211> 78
<212> PRT
<213> Artificial Sequence

<220>

<223> N-terminal peptide of alpha-gliadin

<400> 21
Val Arg Val Pro Val Pro Gln Leu Gln Pro Gln Asn Pro Ser Gln Gln
1               5                   10                  15
Gln Pro Gln Glu Gln Val Pro Leu Val Gln Gln Gln Gln Phe Pro Gly
            20                  25                  30
Gln Gln Gln Gln Phe Pro Pro Gln Gln Pro Tyr Pro Gln Pro Gln Pro
        35                  40                  45
Phe Pro Ser Gln Gln Pro Tyr Leu Gln Leu Gln Pro Phe Pro Gln Pro
    50                  55                  60
Gln Pro Phe Pro Pro Gln Leu Pro Tyr Pro Gln Pro Gln Ser
65                  70                  75

<210> 22
<211> 12
<212> PRT
<213> Artificial Sequence

<220>
<223> N-terminal peptide of alpha-gliadin

<400> 22
Val Arg Val Pro Val Pro Gln Leu Gln Pro Gln Asn
1               5                   10

<210> 23
<211> 20
<212> PRT
<213> Artificial Sequence

<220>
<223> N-terminal peptide of alpha-gliadin

<400> 23
Pro Leu Val Gln Gln Gln Gln Phe Pro Gly Gln Gln Gln Gln Phe Pro
1               5                   10                  15
Pro Gln Gln Pro
            20

<210> 24
<211> 12
<212> PRT
<213> Artificial Sequence

<220>
<223> N-terminal peptide of alpha-gliadin

<400> 24
Gln Pro Tyr Leu Gln Leu Gln Pro Phe Pro Gln Pro
1               5                   10

<210> 25
<211> 14
<212> PRT
<213> Artificial Sequence

<220>
<223> N-terminal peptide of alpha-gliadin

<400> 25
Gln Ser Phe Pro Pro Gln Gln Pro Tyr Pro Gln Gln Gln Pro
1               5                   10

<210> 26
<211> 20
<212> PRT
<213> Artificial Sequence

<220>
<223> N-terminal peptide of alpha-gliadin

<400> 26
Gln Gln Gln Pro Gln Tyr Leu Gln Pro Gln Gln Pro Ile Ser Gln Gln
1               5                   10                  15
Gln Ala Gln Gln
            20

<210> 27
<211> 10
<212> PRT
<213> Artificial Sequence

<220>
<223> N-terminal peptide of alpha-gliadin

<400> 27
Gln Pro Ile Ser Gln Gln Gln Ala Gln Gln
1               5                   10

<210> 28
<211> 16
<212> PRT
<213> Artificial Sequence

<220>
<223> N-terminal peptide of alpha-gliadin

<400> 28
Tyr Pro Gln Pro Gln Ser Phe Pro Pro Gln Gln Pro Tyr Pro Gln Gln
1               5                   10                  15

<210> 29
<211> 12
<212> PRT
<213> Artificial Sequence

<220>
<223> N-terminal peptide of alpha-gliadin

<400> 29
Gln Ser Phe Pro Pro Gln Gln Pro Tyr Pro Gln Gln
1               5                   10

<210> 30
<211> 10

<212> PRT
<213> Artificial Sequence


<220>
<223> N-terminal peptide of alpha-gliadin

<400> 30
Gln Gln Gln Gln Gln Gln Gln Ile Leu Gln
1               5                   10

<210> 31
<211> 10
<212> PRT
<213> Artificial Sequence


<220>
<223> N-terminal peptide of alpha-gliadin

<400> 31
Pro Leu Val Gln Gln Gln Gln Phe Pro Gly
1               5                   10

<210> 32
<211> 24
<212> PRT
<213> Artificial Sequence


<220>
<223> N-terminal peptide of alpha-gliadin

<400> 32
Phe Pro Pro Gln Leu Pro Tyr Pro Gln Pro Gln Ser Phe Pro Pro Gln
1               5                   10                  15
Gln Pro Tyr Pro Gln Gln Gln Pro
            20

<210> 33
<211> 22
<212> PRT
<213> Artificial Sequence


<220>
<223> N-terminal peptide of alpha-gliadin

<400> 33
Phe Pro Pro Gln Leu Pro Tyr Pro Gln Pro Gln Ser Phe Pro Pro Gln
1               5                   10                  15
Gln Pro Tyr Pro Gln Gln
            20

<210> 34
<211> 20
<212> PRT
<213> Artificial Sequence


<220>
<223> N-terminal peptide of gamma-gliadin

<400> 34
Leu Ser Gln Gln Pro Gln Gln Thr Phe Pro Gln Pro Gln Gln Thr Phe
1               5                   10                  15
Pro His Gln Pro
            20

<210> 35
<211> 10
<212> PRT
<213> Artificial Sequence

<220>
<223> N-terminal peptide of gamma-gliadin

<400> 35
Leu Ser Gln Gln Pro Gln Gln Thr Phe Pro
1               5                   10

<210> 36
<211> 82
<212> PRT
<213> Artificial Sequence

<220>
<223> N-terminal peptide of gamma-gliadin

<400> 36
Pro Gln Gln Pro Gln Gln Pro Phe Leu Gln Pro Gln Phe Pro Gln Gln
1               5                   10                  15
Pro Gln Gln Pro Phe Pro Gln Thr Gln Gln Pro Gln Gln Pro Phe Pro
            20                  25                  30
Gln Gln Pro Gln Gln Pro Phe Pro Gln Thr Gln Gln Pro Gln Gln Pro
        35                  40                  45
Phe Pro Gln Gln Pro Gln Gln Pro Phe Pro Gln Thr Gln Gln Pro Gln
    50                  55                  60
Gln Pro Phe Pro Gln Leu Gln Gln Pro Gln Gln Pro Phe Pro Gln Pro
65                  70                  75                  80
Gln Gln

<210> 37
<211> 24
<212> PRT
<213> Artificial Sequence

<220>
<223> N-terminal peptide of gamma-gliadin

<400> 37
Pro Gln Gln Pro Gln Gln Pro Phe Leu Gln Pro Gln Phe Pro Gln Gln
1               5                   10                  15
Pro Gln Gln Pro Phe Pro Gln Thr
            20

<210> 38
<211> 8
<212> PRT
<213> Artificial Sequence

<220>
<223> N-terminal peptide of gamma-gliadin

<400> 38
Pro Gln Gln Pro Phe Pro Gln Gln
1               5

<210> 39
<211> 24
<212> PRT
<213> Artificial Sequence


<220>
<223> N-terminal peptide of gamma-gliadin

<400> 39
Gln Pro Phe Pro Gln Thr Gln Gln Pro Gln Gln Pro Phe Pro Gln Gln
1               5                   10                  15
Pro Gln Gln Pro Phe Pro Gln Thr
            20

<210> 40
<211> 20
<212> PRT
<213> Artificial Sequence


<220>
<223> N-terminal peptide of gamma-gliadin

<400> 40
Pro Gln Gln Pro Phe Pro Gln Leu Gln Gln Pro Gln Gln Pro Phe Pro
1               5                   10                  15
Gln Pro Gln Gln
            20

<210> 41
<211> 12
<212> PRT
<213> Artificial Sequence


<220>
<223> N-terminal peptide of gamma-gliadin

<400> 41
Pro Gln Gln Pro Gln Gln Ser Phe Pro Gln Gln Gln
1               5                   10

<210> 42
<211> 78
<212> PRT
<213> Artificial Sequence


<220>
<223> N-terminal peptide of gamma-gliadin

<400> 42
Pro Phe Leu Gln Pro Gln Gln Pro Phe Pro Gln Gln Pro Gln Gln Pro
1               5                   10                  15
Phe Pro Gln Thr Gln Gln Pro Gln Gln Pro Phe Pro Gln Gln Pro Gln

20                          25                          30
Gln Pro Phe Pro Gln Thr Gln Gln Pro Gln Gln Pro Phe Pro Gln Gln
            35                          40                          45
Pro Gln Gln Pro Phe Pro Gln Thr Gln Gln Pro Gln Gln Pro Phe Pro
    50                          55                          60
Gln Leu Gln Gln Pro Gln Gln Pro Phe Pro Gln Pro Gln Gln
65                          70                          75

<210> 43
<211> 10
<212> PRT
<213> Artificial Sequence

<220>
<223> N-terminal peptide of gamma-gliadin

<400> 43
Leu Gln Pro Gln Gln Pro Phe Pro Gln Gln
1               5                   10

<210> 44
<211> 12
<212> PRT
<213> Artificial Sequence

<220>
<223> N-terminal peptide of gamma-gliadin

<400> 44
Gln Pro Gln Gln Ser Phe Pro Gln Gln Gln Arg Pro
1               5                   10

<210> 45
<211> 10
<212> PRT
<213> Artificial Sequence

<220>
<223> N-terminal peptide of gamma-gliadin

<400> 45
Gln Pro Gln Gln Ser Phe Pro Gln Gln Gln
1               5                   10

<210> 46
<211> 28
<212> PRT
<213> Artificial Sequence

<220>
<223> N-terminal peptide of gamma-gliadin

<400> 46
Leu Ser Gln Gln Pro Gln Gln Thr Phe Pro Gln Pro Gln Gln Thr Phe
1               5                   10                  15
Pro His Gln Pro Gln Gln Gln Val Pro Gln Pro Gln
            20                  25

<210> 47

<211> 26
<212> PRT
<213> Artificial Sequence

<220>
<223> N-terminal peptide of gamma-gliadin

<400> 47
Leu Ser Gln Gln Pro Gln Gln Thr Phe Pro Gln Pro Gln Gln Thr Phe
1               5                   10                  15
Pro His Gln Pro Gln Gln Gln Val Pro Gln
            20                  25

<210> 48
<211> 102
<212> PRT
<213> Artificial Sequence

<220>
<223> N-terminal peptide of gamma-gliadin

<400> 48
Gln Pro Gln Gln Pro Phe Leu Gln Pro Gln Gln Pro Phe Pro Gln Gln
1               5                   10                  15
Pro Gln Gln Pro Phe Pro Gln Thr Gln Gln Pro Gln Gln Pro Phe Pro
            20                  25                  30
Gln Gln Pro Gln Gln Pro Phe Pro Gln Thr Gln Gln Gln Gln Pro Gln
        35                  40                  45
Gln Pro Phe Pro Gln Gln Pro Gln Gln Pro Phe Pro Gln Thr Gln Gln
    50                  55                  60
Pro Gln Gln Pro Phe Pro Gln Leu Gln Gln Pro Gln Gln Pro Phe Pro
65                  70                  75                  80
Gln Pro Gln Gln Gln Leu Pro Gln Pro Gln Gln Pro Gln Gln Ser Phe
                85                  90                  95
Pro Gln Gln Gln Arg Pro
            100

<210> 49
<211> 12
<212> PRT
<213> Artificial Sequence

<220>
<223> N-terminal peptide of gamma-gliadin

<400> 49
Leu Gln Pro Gln Gln Pro Phe Pro Gln Gln Pro Gln
1               5                   10

<210> 50
<211> 10
<212> PRT
<213> Artificial Sequence

<220>
<223> N-terminal peptide of gamma-gliadin

<400> 50
Leu Gln Pro Gln Gln Pro Phe Pro Gln Gln

1                   5                   10

<210> 51
<211> 4
<212> PRT
<213> Artificial Sequence


<220>
<223> N-terminal peptide of gamma-gliadin

<400> 51
Gln Thr Gln Gln
1

<210> 52
<211> 4
<212> PRT
<213> Artificial Sequence


<220>
<223> N-terminal peptide of gamma-gliadin

<400> 52
Gln Gln Pro Gln
1

<210> 53
<211> 18
<212> PRT
<213> Artificial Sequence


<220>
<223> N-terminal peptide of gamma-gliadin

<400> 53
Phe Pro Gln Thr Gln Gln Gln Gln Pro Gln Gln Pro Phe Pro Gln Gln
1               5                   10                  15
Pro Gln


<210> 54
<211> 26
<212> PRT
<213> Artificial Sequence


<220>
<223> N-terminal peptide of gamma-gliadin

<400> 54
Phe Pro Gln Thr Gln Gln Pro Gln Gln Pro Phe Pro Gln Leu Gln Gln
1               5                   10                  15
Pro Gln Gln Pro Phe Pro Gln Pro Gln Gln
            20                  25

<210> 55
<211> 10
<212> PRT
<213> Artificial Sequence

<220>
<223> N-terminal peptide of gamma-gliadin

<400> 55
Gln Pro Gln Gln Ser Phe Pro Gln Gln Gln
1               5                   10

<210> 56
<211> 20
<212> PRT
<213> Artificial Sequence


<220>
<223> N-terminal peptide of gamma-gliadin

<400> 56
Leu Ser Gln Gln Pro Gln Gln Thr Phe Pro Gln Pro Gln Gln Thr Phe
1               5                   10                  15
Pro His Gln Pro
            20

<210> 57
<211> 10
<212> PRT
<213> Artificial Sequence


<220>
<223> N-terminal peptide of gamma-gliadin

<400> 57
Gln Gln Pro Gln Gln Thr Phe Pro Gln Pro
1               5                   10

<210> 58
<211> 14
<212> PRT
<213> Artificial Sequence


<220>
<223> N-terminal peptide of gamma-gliadin

<400> 58
Pro Gln Pro Gln Gln Pro Gln Gln Ser Phe Pro Gln Gln Gln
1               5                   10

<210> 59
<211> 12
<212> PRT
<213> Artificial Sequence


<220>
<223> N-terminal peptide of gamma-gliadin

<400> 59
Pro Gln Gln Pro Gln Gln Ser Phe Pro Gln Gln Gln
1               5                   10

<210> 60

<211> 10
<212> PRT
<213> Artificial Sequence

<220>
<223> N-terminal peptide of gamma-gliadin

<400> 60
Gln Pro Gln Gln Ser Phe Pro Gln Gln Gln
1               5                   10

<210> 61
<211> 76
<212> PRT
<213> Artificial Sequence

<220>
<223> N-terminal peptide of gamma-gliadin

<400> 61
Leu Gln Pro Gln Gln Pro Phe Pro Gln Gln Pro Gln Gln Pro Phe Pro
1               5                   10                  15
Gln Thr Gln Gln Pro Gln Gln Pro Phe Pro Gln Gln Pro Gln Gln Pro
            20                  25                  30
Phe Pro Gln Thr Gln Gln Pro Gln Gln Pro Phe Pro Gln Gln Pro Gln
        35                  40                  45
Gln Pro Phe Pro Gln Thr Gln Gln Pro Gln Gln Pro Phe Pro Gln Leu
    50                  55                  60
Gln Gln Pro Gln Gln Pro Phe Pro Gln Pro Gln Gln
65                  70                  75

<210> 62
<211> 12
<212> PRT
<213> Artificial Sequence

<220>
<223> N-terminal peptide of gamma-gliadin

<400> 62
Leu Gln Pro Gln Gln Pro Phe Pro Gln Gln Pro Gln
1               5                   10

<210> 63
<211> 12
<212> PRT
<213> Artificial Sequence

<220>
<223> N-terminal peptide of gamma-gliadin

<400> 63
Gln Gln Pro Gln Gln Pro Phe Pro Gln Gln Pro Gln
1               5                   10

<210> 64
<211> 12
<212> PRT
<213> Artificial Sequence

<220>
<223> N-terminal peptide of gamma-gliadin

<400> 64
Gln Gln Pro Gln Gln Pro Phe Pro Gln Pro Gln Gln
1               5                   10

<210> 65
<211> 10
<212> PRT
<213> Artificial Sequence


<220>
<223> N-terminal peptide of alpha-gliadin

<400> 65
Gln Glu Gln Val Pro Leu Val Gln Gln Gln
1               5                   10

<210> 66
<211> 10
<212> PRT
<213> Artificial Sequence


<220>
<223> N-terminal peptide of alpha-gliadin

<400> 66
Val Gln Gln Gln Gln Phe Pro Gly Gln Gln
1               5                   10

<210> 67
<211> 10
<212> PRT
<213> Artificial Sequence


<220>
<223> N-terminal peptide of alpha-gliadin

<400> 67
Gln Phe Pro Gly Gln Gln Gln Gln Phe Pro
1               5                   10

<210> 68
<211> 10
<212> PRT
<213> Artificial Sequence


<220>
<223> N-terminal peptide of alpha-gliadin

<400> 68
Pro Gly Gln Gln Gln Gln Phe Pro Pro Gln
1               5                   10

<210> 69
<211> 10

<212> PRT
<213> Artificial Sequence

<220>
<223> N-terminal peptide of alpha-gliadin

<400> 69
Gln Gln Gln Gln Phe Pro Pro Gln Gln Pro
1               5                   10

<210> 70
<211> 10
<212> PRT
<213> Artificial Sequence

<220>
<223> N-terminal peptide of alpha-gliadin

<400> 70
Pro Gln Gln Pro Tyr Pro Gln Pro Gln Pro
1               5                   10

<210> 71
<211> 10
<212> PRT
<213> Artificial Sequence

<220>
<223> N-terminal peptide of alpha-gliadin

<400> 71
Gln Pro Phe Pro Ser Gln Gln Pro Tyr Leu
1               5                   10

<210> 72
<211> 10
<212> PRT
<213> Artificial Sequence

<220>
<223> N-terminal peptide of alpha-gliadin

<400> 72
Phe Pro Ser Gln Gln Pro Tyr Leu Gln Leu
1               5                   10

<210> 73
<211> 10
<212> PRT
<213> Artificial Sequence

<220>
<223> N-terminal peptide of alpha-gliadin

<400> 73
Ser Gln Gln Pro Tyr Leu Gln Leu Gln Pro
1               5                   10

<210> 74
<211> 10
<212> PRT
<213> Artificial Sequence

<220>
<223> N-terminal peptide of alpha-gliadin

<400> 74
Tyr Pro Gln Gln Gln Pro Gln Tyr Leu Gln
1               5                   10

<210> 75
<211> 10
<212> PRT
<213> Artificial Sequence

<220>
<223> N-terminal peptide of alpha-gliadin

<400> 75
Gln Ile Leu Gln Gln Ile Leu Gln Gln Gln
1               5                   10

<210> 76
<211> 10
<212> PRT
<213> Artificial Sequence

<220>
<223> N-terminal peptide of alpha-gliadin

<400> 76
Gln Ile Leu Gln Gln Gln Leu Ile Pro Cys
1               5                   10

<210> 77
<211> 10
<212> PRT
<213> Artificial Sequence

<220>
<223> N-terminal peptide of alpha-gliadin

<400> 77
Gln Val Pro Leu Val Gln Gln Gln Gln Phe
1               5                   10

<210> 78
<211> 10
<212> PRT
<213> Artificial Sequence

<220>
<223> N-terminal peptide of gamma-gliadin

<400> 78
Leu Ala Gln Ile Pro Gln Gln Leu Gln Cys

1               5                   10

<210> 79
<211> 10
<212> PRT
<213> Artificial Sequence

<220>
<223> N-terminal peptide of gamma-gliadin

<400> 79
Gln Gln Gln Leu Pro Gln Pro Gln Gln Pro
1               5                   10

<210> 80
<211> 10
<212> PRT
<213> Artificial Sequence

<220>
<223> N-terminal peptide of gamma-gliadin

<400> 80
Leu Gln Gln Gln Leu Val Pro Gln Leu Gln
1               5                   10

<210> 81
<211> 10
<212> PRT
<213> Artificial Sequence

<220>
<223> N-terminal peptide of gamma-gliadin

<400> 81
Gln Gln Leu Val Pro Gln Leu Gln Gln Pro
1               5                   10

<210> 82
<211> 10
<212> PRT
<213> Artificial Sequence

<220>
<223> N-terminal peptide of gamma-gliadin

<400> 82
Gln Gln Ser Phe Pro Gln Gln Gln Arg Pro
1               5                   10

<210> 83
<211> 10
<212> PRT
<213> Artificial Sequence

<220>
<223> N-terminal peptide of gamma-gliadin

<400> 83
Gln Thr Gln Gln Pro Gln Gln Pro Phe Pro
1               5                   10

<210> 84
<211> 28
<212> DNA
<213> Artificial Sequence

<220>
<223> primer for the cloning of the C-terminal domain of alpha-gliadin

<400> 84
cacggatcca tgcagggatg tcgtcttg                                          28

<210> 85
<211> 31
<212> DNA
<213> Artificial Sequence

<220>
<223> primer for the cloning of the C-terminal domain of alpha-gliadin

<400> 85
ctcgagttat gagttagtac cgaagatgcc a                                      31

<210> 86
<211> 18
<212> DNA
<213> Artificial Sequence

<220>
<223> primer for the cloning of recombinant gamma-gliadin

<400> 86
cgatggcaac aactatcg                                                     18

<210> 87
<211> 18
<212> DNA
<213> Artificial Sequence

<220>
<223> primer for the cloning of recombinant gamma-gliadin

<400> 87
tcattgtcca ctgatgcc                                                     18

<210> 88
<211> 27
<212> DNA
<213> Artificial Sequence

<220>

<223> primer for the cloning of recombinant gamma-gliadin

<400> 88
caccatgaat atgcaggtcg accctag                                          27


<210> 89
<211> 27
<212> DNA
<213> Artificial Sequence


<220>
<223> primer for the cloning of recombinant C-terminal domain of
      gamma-gliadin

<400> 89
actccatggc ttgcaagaat tacctct                                          27


<210> 90
<211> 30
<212> DNA
<213> Artificial Sequence


<220>
<223> primer for the cloning of recombinant C-terminal domain of
      gamma-gliadin

<400> 90
gtgctcgagt ccactgatgc cggcgactat                                       30


<210> 91
<211> 27
<212> DNA
<213> Artificial Sequence


<220>
<223> primer for the cloning of recombinant N-terminal domain of
      gamma-gliadin

<400> 91
caccatgaat atgcaggtcg accctag                                          27


<210> 92
<211> 23
<212> DNA
<213> Artificial Sequence


<220>
<223> primer for the cloning of recombinant N-terminal domain of
      gamma-gliadin

<400> 92
ttgtagatat ggctgaatca acg                                              23

## Description

### DOMAIN OF THE INVENTION

The present invention relates to an immunogenic polypeptide for use as a medicament.

In particular, said immunogenic polypeptide comprises the N-terminal domain of an α-gliadin or a γ-gliadin, or a fragment of the N-terminal domain of an α-gliadin or a γ-gliadin, and is for use as an agent for desensitization of an individual with diagnosed IgE-dependent gluten allergy.

### BACKGROUND OF THE INVENTION

To date, the prevalence of food allergy is about 2% of the adult population, whereas the incidence in children is higher and accounts for 3-7% in this subpopulation. The majority of children outgrow food allergies by the age of three.

In food allergies, the body produces immunoglobulin E (IgE) antibodies, which react with the food-borne allergen and subsequently trigger a reaction with the mast cells, in tissues, and basophils, in the blood cell. Mast cells are found below the surface of the skin and in the membranes lining the nose, respiratory tract, eyes and intestine. A substance called histamine or other substances such as leukotrienes and prostaglandins are released from mast cells and basophils subsequently causing allergic responses such as (i) a respiratory response, such as runny nose or nasal congestion, sneezing, asthma, coughing, wheezing, breathing difficulties; (ii) a skin response, such as swelling of the lips, mouth, tongue, face and/or throat (angio-oedema), hives, rashes or redness, pruritus, eczema; (iii) a gastrointestinal response, such as abdominal cramps, diarrhoea, nausea, vomiting, colic, bloating; and/or (iv) a systemic response, namely an anaphylactic shock (severe generalised shock).

Fortunately, most allergic reactions to food are relatively mild but a small number of people experience a severe and life-threatening reaction called anaphylaxis. An anaphylactic reaction can sometimes occur within a few minutes of exposure and immediate medical attention is necessary.

In westernized countries, gluten allergies are among the tenth most prevalent food allergies, after cow's milk or peanut allergies to name a few. Gluten consists in an admixture of proteins belonging to the prolamin superfamily, such as gliadins and glutenins.

The prolamin superfamily comprises the largest number of allergenic plant proteins. It includes proteins such as the sulphur-rich prolamins, the seed storage 2S albumins, the nonspecific lipid transfer proteins (LTP) and the cereal α-amylase/ trypsin inhibitors (ATI) that are involved in food or respiratory allergies. Prolamin superfamily members are grouped on the basis on a conserved skeleton of 8 cysteine residues that are connected by intramolecular disulphide bonds. In LTPs, 2S albumins and ATIs, these disulphide bonds contribute significantly to the stabilization of a compact, α-helix-rich 3D structure that is resistant to thermal and enzymatic denaturation and plays an essential role in the allergenic potential of these proteins.

The sulphur-rich prolamins, α-gliadins, γ-gliadins and low molecular weight (LMW) glutenin subunits differ from the other members of the prolamin superfamily by their organization into two structural domains: the C-terminal (Ct-) domain, which include the cysteine skeleton, and the N-terminal (Nt-) domain, which contains repeating motifs that are rich in glutamine and proline residues. To date, no 3D structure of prolamins has been determined. Sulphur-rich prolamins are considered to be extended molecules, though their Ct-domain is assumed to be more compact than the Nt- domain due to the disulphide bonds. Studies of the secondary structures of these proteins showed that α- and γ-gliadins contain approximately 30% or more α-helical structures.

Many α- and γ-gliadin peptides have also been identified as T-cell epitopes in celiac disease, with most of them being from the repetitive domain (Camarca et al.; J. Immunol. 2009, 182, 4158-4166). Senger et al. (J. Immunol. 2005, 175, 8087-8095) identified 2 immunodominant epitopes borne by α-gliadin and their potential uses for desensitisation of celiac disease. Maurano et al. (Scand. J. immunol. 2001, 53, 290-295) observed that a purified fraction of α-gliadin is capable of suppressing the immune response that takes place in individual having the celiac disease. WO 2003/104273 discloses a method of desensitisation as a treatment of the celiac disease, based on fragments of N-terminal domain of α-gliadin. Finally, Huibregtse et al. (J. Immunol. 2009, 183, 2390-2396) discloses the identification of an epitope borne by the C-terminal domain of α-gliadin, which may be involved in tolerance.

However, it is needless to mention that the physiological mechanisms involved in the celiac disease and the wheat allergies are distinct.

Indeed, the celiac disease is an inflammatory disease, wherein the T cells are activated upon recognition of the epitopes borne by the gliadine proteins, through the MHC complex. In other words, the celiac disease encompasses an immune response that is IgE-independent.

By contrast, the wheat allergy is mediated by blood circulating IgEs.

Therefore, the epitopes involved in celiac diseases are necessarily distinct from the epitopes involved in wheat allergy, although overlapping may be observed.

With respect to wheat allergies, much attention has been paid on ω5-gliadins because of their role in wheat-dependent exercise-induced anaphylaxis. However, α- and γ-gliadins have also been identified as IgE-binding proteins in several studies (Battais et al.; Clin. Exp. Allergy 2003, 33, 962-970; Battais et al.; J. Cereal Sci. 2005, 42, 109-117.Hofmann et al.; Allergy 2012, 67, 1457-1460; Mäkelä et al.; Clin. Exp. Allergy 2014, 44, 1420-1430; Waga et al.; Am. J. Plant. Sci. 2011, 2, 476-483; and Yokooji et al.; Allergol. Int., 2013, 62(4), 435-445).

A minor role of α-gliadin was also reported in bakers with occupational asthma (Bittner et al.; J. Allergy Clin. Immunol. 2008, 121, 744-749; Sander et al. Allergy 2011, 66, 1208-1215). IgE-binding epitopes of gliadins were primarily found in the repetitive domains of these proteins (Denery-Papini et al.; Clin. Exp. Allergy 2011, 41, 1478-1492, Battais et al.; Allergy 2005, 60, 815-821; Matsuo et al.; FEBS J. 2005, 272, 4431-4438; Tanabe; J. Nutr. Sci. Vitaminol. 2004, 50, 367-370). This different antigenicity of the two gliadin structural domains may be related to their asymmetric organization. However, if fewer continuous epitopes were identified in the Ct-domains of α- and γ-gliadins, several of them contain a cysteine or are in the vicinity of a cysteine, indicating that they might participate in discontinuous epitopes.

Wheat gliadins present a very large intra- and inter-cultivar polymorphism; thus, each gliadin sub-group contains many homologous proteins (Dupont et al.; Proteome Sci. 2011, 9, 10; Metakovsky et al.; J. Genet. Breed 1991, 45, 325-344). Purifying individual natural gliadins is thus very difficult. To overcome these problems, recombinant proteins are an alternative way. Some recombinant prolamin allergens have already been produced and characterized (Bittner et al.; J. Allergy Clin. Immunol. 2008, 121, 744-749; Sander et al. Allergy 2011, 66, 1208-1215, Baar et al.; J. Immunol. 2012, 189, 3018-3025; Baar et al.; Allergy, 2014, 69, 1316-1323; Mameri et al.; J. of Agric. Food Chem., 2012, 60, 7538-7547; Mameri et al.; J. Agric. Food Chem., 2012, 60, 8059-8068; Matsuo et al.; FEBS J., 2005, 272, 4431-4438). Well-defined protein fragments can also be produced by heterologous expression, and such recombinant fragments have been valuable for studying the antigenicity of LMW glutenin subunits and their two domains (Mameri et al.; J. of Agric. Food Chem., 2012, 60, 7538-7547) as well as fragments of HMW glutenins (Baar et al.; Allergy, 2014, 69, 1316-1323).

Unfortunately, no efficient therapy for desensitization of individuals with gluten allergy has been reported to date, which is largely due to the allergic properties of these proteins which prevent their use as antigens. Therefore, individuals with gluten allergy have to avoid gluten containing food, which food regimen may very compelling, as gluten is found in a vast number of food products. In another alternative, the food industry managed to develop gluten products wherein the gluten proteins are deaminated. However, the deamination process is time and money consuming, which in turn result in deaminated gluten products that are more expensive for the consumers.

Hence, there is a need for providing a tool for use in the desensitization of individuals with IgE-dependent gluten allergy specifically implying gliadins.

### SUMMARY OF THE INVENTION

In one aspect, the invention relates to an immunogenic composition comprising:
- a polypeptide or a mixture of polypeptides comprising the N-terminal domain of an α-gliadin or a γ-gliadin, or a fragment of the N-terminal domain of an α-gliadin or a γ-gliadin; and
- at least one physiologically acceptable adjuvant.

In another aspect, the invention also relates to an immunogenic composition or polypeptide according to the invention, for use as a medicament.

In another aspect, the invention also relates to an immunogenic composition or polypeptide according to the invention, for use as an antigenic compound.

In another aspect, the invention also relates to an immunogenic composition or polypeptide according to the invention, for use as an agent for desensitization of individual with an IgE-dependent gluten allergy.

In another aspect, the invention also relates to a method for desensitization of an individual with an IgE-dependent gluten allergy comprising a step of administering to said individual an immunogenic composition comprising a polypeptide comprising a N-terminal domain of an α-gliadin or a γ-gliadin, or a fragment thereof, optionally linked to a carrier molecule.

### LEGENDS OF THE FIGURES

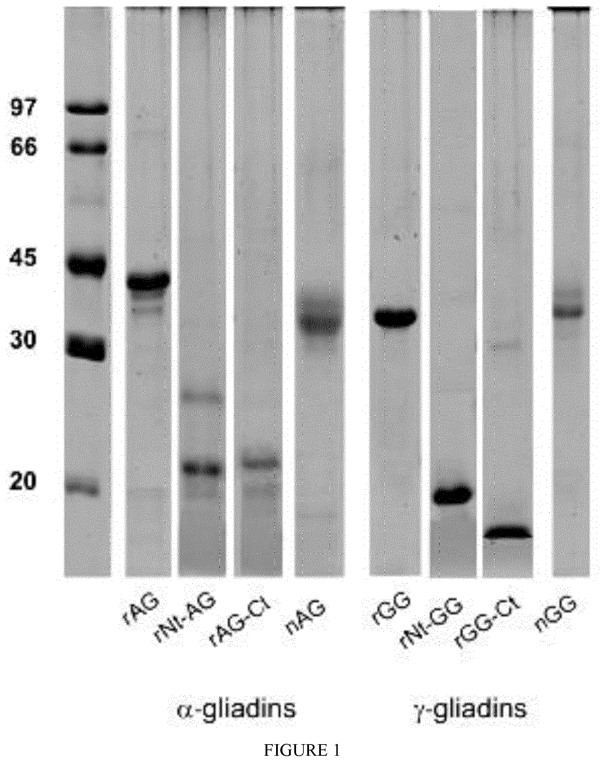

**Figure 1****:** Illustration of a SDS-PAGE analysis of the proteins under reducing conditions coloured with Coomassie Brilliant Blue R250. "rAG" (SEQ ID No. 3) and "rGG" (SEQ ID No. 4) are for recombinant α-gliadin and γ-gliadin respectively; "rNt-AG" (SEQ ID No. 5) and "rNt-GG" (SEQ ID No. 6) are respectively for recombinant Nt-domains of α-gliadin and γ-gliadin; "rAG-Ct" (SEQ ID No. 7), "rGG-Ct" (SEQ ID No. 8) are respectively for Ct- domains of α-gliadin and γ-gliadin; "nAG" (SEQ ID No. 1) and "nGG" (SEQ ID No. 2) are for respectively natural α-gliadin and γ-gliadin.

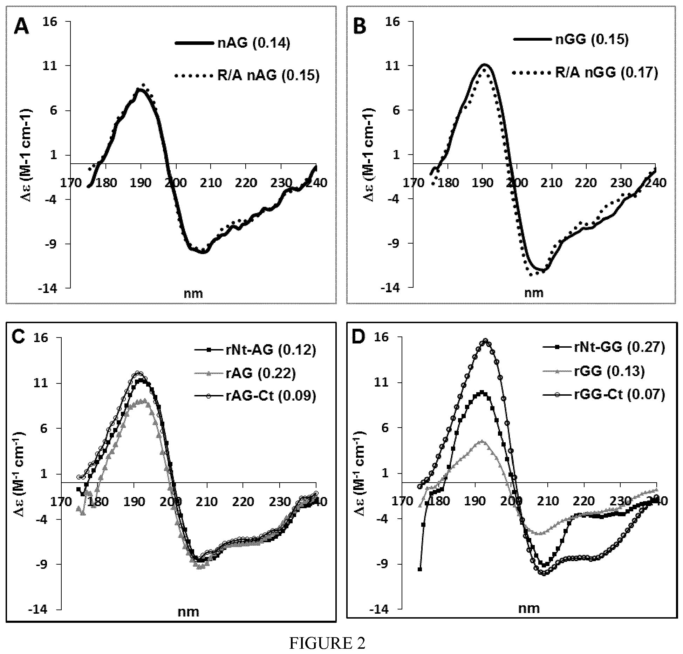

**Figure 2****:** Plots displaying the Synchrotron radiation circular dichroism (SRCD) analysis on natural and recombinant gliadins. Spectra of native (plain line) and reduced alkylated (R/A, dash line) natural α-gliadin (nAG; SEQ ID No. 1) (**A**); natural γ-gliadin (nGG; SEQ ID No. 2) (**B**). Spectra of recombinant full-length (triangles), N-terminal (Nt; squares) and C-terminal (Ct, diamonds) domains of rAG (**C**) and rGG (**D**). Normalized root-mean-square deviations (NRMSD) are indicated in parentheses.

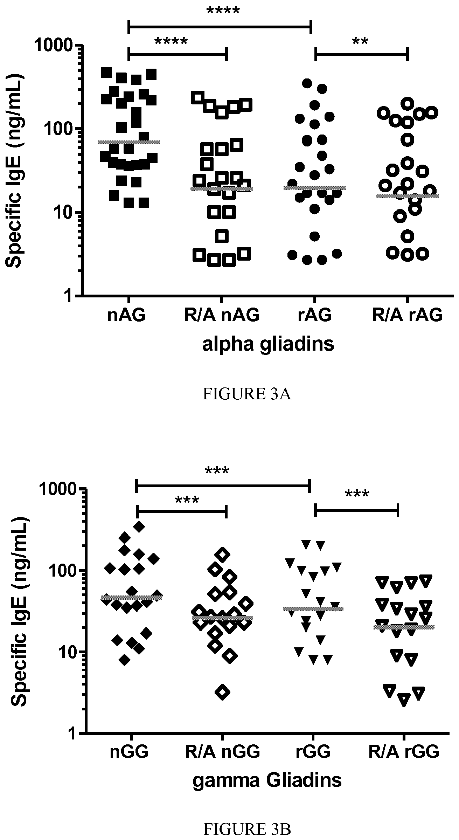

**Figure 3****:** Graphs illustrating the effect of heterologous expression and reduction/alkylation of natural and recombinant α-gliadins (A) and γ-gliadins (B) on IgE binding measured by ELISA. Means and medians per sample are indicated in Tables 1 and 2. Medians, plotted as grey bars, are significantly different (** : 0.001< p<0.01; ***: 0.0001<p<0.001; ****: p<0.0001, Wilcoxon signed rank test, n=28 for AG and n=20 for GG). Fifteen data (5 for R/A nAG (SEQ ID No. 1); 3 for rAG (SEQ ID No. 3); 7 for R/A rAG (SEQ ID No. 3)) and seven data (2 for R/A nGG (SEQ ID No. 2); 2 for rGG (SEQ ID No. 4); 3 for R/A rGG (SEQ ID No. 4)) below the detection limit are not drawn on the graphs.

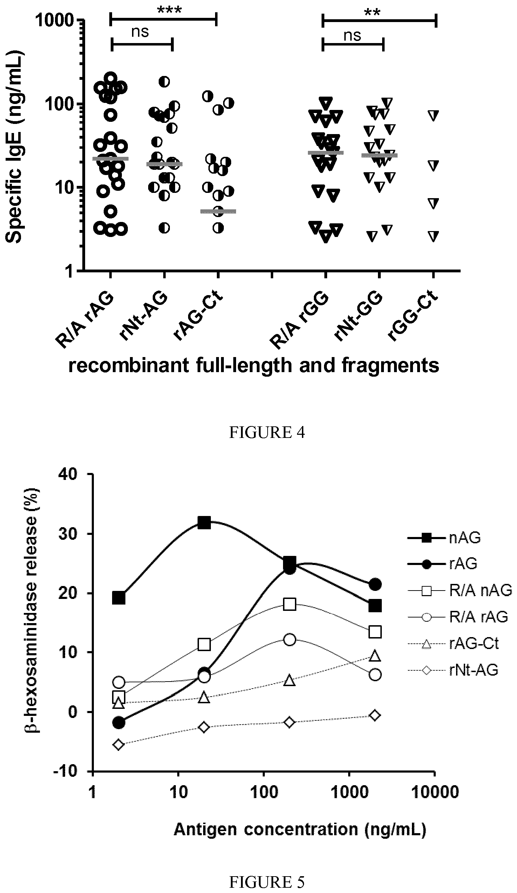

**Figure 4****:** Graph illustrating the comparison of IgE binding to the full-length gliadins and to their N-terminal (Nt) or C-terminal (Ct) domains measured by ELISA. Means and medians per sample are indicated in Table 3. Statistical differences between medians of specified groups using Friedman and Dunn's multiple comparison post-test are indicated. Medians, plotted as grey bars, are significantly different (**: 0.001< p<0.01; ***: p<0.001; n=21 for AG and 17 for GG) or not (ns: p>0.05). Twenty-four data (2 for rNt-AG (SEQ ID No. 5); 9 for rAG-Ct (SEQ ID No. 7) and 13 for rGG-Ct (SEQ ID No. 8)) below the detection limit are not drawn on the graph.
**Figure 5****:** Graph illustrating the triggering potential of natural and recombinant α-gliadins and Nt-domain (SEQ ID No. 5) or Ct-domain (SEQ ID No. 7) tested in an Rat basophil leukaemia cell degranulation assay with a pool of 2 sera. Duplicate measurements were performed and means are represented on the graph. Abbreviations are identical to those in Figures 1 and 2. Closed squares are for natural α-gliadin; closed circles are for recombinant α-gliadin; open squares are for reduced alkylated natural α-gliadin; open circles are for reduced alkylated recombinant α-gliadin; open triangles are for recombinant C-terminal domain of α-gliadin; open diamonds are for recombinant N-terminal domain of α-gliadin.

### DETAILED DESCRIPTION OF THE INVENTION

### Therapeutic Indication

The invention relies upon the finding, by the inventors, that polypeptides comprising a N-terminal domain of an α-gliadin or a γ-gliadin, or a fragment thereof may be for use as a medicament, and more precisely as antigens for desensitization of individuals affected with an allergy to gluten.

As shown in the examples herein, the inventors have experimentally shown that a polypeptide comprising the N-terminal domain of an α-gliadin is able to elicit IgE-binding, without however eliciting efficient basophils degranulation, which is normally occurring during the course of an allergic response in gluten allergy.

As mentioned above, both α-gliadins and γ-gliadins are proteins organized as a two-domain structure. The N-terminal (Nt-) domain mainly comprises repeating motifs that are rich in glutamine and proline residues, whereas the C-terminal (Ct-) domain includes a cysteine skeleton.

Within the scope of the present invention, the expression "N-terminal domain of an α-gliadin or a γ-gliadin" encompasses the region of amino acids residues belonging to the N-terminal domain of an α-gliadin or a γ-gliadin and may comprise at most one cysteine residue. In other words, the "N-terminal domain of an α-gliadin or a γ-gliadin" consists of the peptide region preceding the 1^{st} cysteine residue, or alternatively the peptide region including at most the 1^{st} cysteine residue, among the numerous cysteine residues found in the linear sequence of a full length α-gliadin or γ-gliadin.

In some embodiments, the "N-terminal domain of an α-gliadin or a γ-gliadin" consists of the peptide region starting from the first amino acid residue at the N-terminus position, including at most the 1^{st} cysteine residue of the linear amino acid residues chain, and further including at most the 7 amino acid residues consecutive to the said 1^{st} cysteine residue.

Within the scope of the present invention, the term "fragment" encompasses any polypeptide having at least 4 consecutive amino acid residues of a full length N-terminal domain of an α-gliadin or a γ-gliadin.

Within the scope of the present invention, the term "medicament" is intended to mean a product comprising a pharmacologically active ingredient intended to be administered to a human or animal body in order to attenuate or to cure symptoms associated with a disease.

### Polypeptide comprised in an immunogenic composition

The polypeptide comprising the N-terminal domain of an α-gliadin or a γ-gliadin, or a fragment thereof may originate from any known plant species having been described for possessing such proteins. In particular, the invention relates to polypeptides comprising the N-terminal domain of an α-gliadin or a γ-gliadin, or fragments thereof originating mainly from wheat, spelt and kamut.

It is commonly accepted in the art that α-gliadins and γ-gliadins present an important polymorphism, within one single species and among several species (Dupont et al.; Proteome Sci. 2011, 9, 10; Metakovsky et al.; J. Genet. Breed 1991, 45, 325-344). It is generally admitted in the art that the percentage of identity between two molecules of α-gliadins or two molecules of γ-gliadins is about 70%. This average percentage of minimal identity is to take into consideration to evaluate the intra-species as well as the inter-species polymorphism.

As used herein, the percent identity between the two sequences is a function of the number of identical positions shared by the sequences (i. e., % identity = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described below.

The percent identity between two amino acid sequences can be determined using the algorithm of E. Meyers and W. Miller (Comput. Appl. Biosci., 4:1 1-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. MoI, Biol. 48:444-453, 1970) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1 , 2, 3, 4, 5, or 6.

Within the scope of the present invention, the expression "a polypeptide having at least 70% amino acid identity" with a reference polypeptide sequence encompasses polypeptides having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 100% identity with the said reference polypeptide.

In some embodiments, a polypeptide comprising the N-terminal domain of an α-gliadin or a γ-gliadin, or a fragment thereof may be for use as an antigenic compound.

Within the scope of the present invention, the expression "antigenic compound" is intended to mean a compound able to elicit an immunologic response, notably through the production of antibodies, when administered in a human or animal body in an appropriate form (e.g. as an immune-conjugate in a composition further comprising an immuno-adjuvant substance). In human, said production of antibodies accounts for the specific production of IgGs.

In some embodiments, a polypeptide comprising the N-terminal domain of an α-gliadin or a γ-gliadin, or a fragment thereof may be for use as an agent for desensitization of an individual with an IgE-dependent gluten allergy.

Within the scope of the present invention, the term "desensitization of an individual with an IgE-dependent gluten allergy" is intended to mean a process during which an individual with an IgE-dependent gluten allergy is stimulated with one single dose or alternatively with gradually increasing doses of the substances to which a person is allergic in order to modify or stop the allergic response.

In some embodiments, said IgE-dependent gluten allergy is select in the group of durum wheat allergy, bread wheat allergy, spelt allergy, kamut allergy.

Notably, wheat (bran, durum, germ, gluten, grass, malt, sprouts, starch), spelt and/or kamut may be found in food products such as bread crumbs, bulgur, cereal extract, club wheat, couscous, cracker meal, einkorn, emmer, farina, flour (all purpose, bread, cake, durum, enriched, graham, high gluten, high protein, instant, pastry, self-rising, soft wheat, steel ground, stone ground, whole wheat), hydrolyzed wheat protein, matzoh (also known as matzo, matzah, or matza), pasta, seitan, semolina, sprouted wheat, triticale, vital wheat gluten, wheat bran hydrolysate, wheat germ oil, wheat grass, wheat protein isolate, whole wheat berries.

Traces of wheat, spelt and/or kamut may also be found in food products such as ale, baking mixes, baked products, batter-fried foods, beer, breaded foods, breakfast cereals, candy, crackers, glucose syrup, hot dogs, ice cream, imitation crabmeat, marinara sauce, play dough, potato chips, processed meats, rice cakes, salad dressings, sauces, soups, soy sauce, starch (gelatinized starch, modified starch, modified food starch, vegetable starch), surimi and turkey patties.

In some embodiments, said IgE-dependent gluten allergy is a cross-allergy between α-gliadin allergy and γ-gliadin allergy.

In some embodiments, said polypeptide is a recombinant polypeptide.

Any routinely method to obtain a recombinant polypeptide according to the invention may be employed by a skilled in the art. A skilled in the art may adapt suitable protocols from the available genetic engineering techniques to provide such recombinant polypeptides. Notably the techniques allowing for DNA recombination, DNA cloning, DNA incorporation into a suitable host, selection of the appropriate promoters are within the skills of an artisan in the art, as well as the techniques allowing for polypeptide purification (see e.g. Green and Sambrook; Molecular Cloning, A Laboratory Manual; 4th Edition, 2012; Cold Spring Harbor Laboratory Press).

In some other embodiments, said polypeptide may be obtained by chemical synthesis, accordingly to methods known in the state in the art. For example, polypeptides according to the invention may be synthesized by the solid-phase synthesis method initially developed by Merrifield (Solid Phase Peptide Synthesis. I. The Synthesis of a Tetrapeptide. J. Am. Chem. Soc. 85 (14): 2149-2154), by the liquid-phase synthesis method (Bayer and Mutter. Nature 237, 512 - 513 (30 June 1972)).

In certain embodiments, the polypeptide according to the invention is a reduced polypeptide.

In other embodiments, the polypeptide according to the invention is an alkylated polypeptide.

In some embodiments, the polypeptide according to the invention is a reduced and/or alkylated polypeptide.

In some preferred embodiments, the polypeptide according to the invention is a reduced and alkylated polypeptide.

The inventors surprisingly found that, as shown in the examples below, reduced and/or alkylated polypeptides of the N-terminal domain of α-gliadin or a γ-gliadin may be of use as an epitope capable of binding to IgEs, without, however, promoting degranulation of basophiles.

Denery et al. (Clin. And Exp. Allergy, 2011, 41, 1478-1492) had observed that lipid transfer protein (LTP1) exposes epitopes that are recognized by patients allergic to wheat. However, reduced and alkylated LTP were poorly recognized by patient IgEs. Therefore, there was a prejudice in the art to take advantage of reduced and/or alkylated epitopes, in particular when these epitopes are intended to be used as agent for desensitize patients that are allergic to wheat.

In practice any method for reducing and/or alkylating a polypeptide known from the skilled in the art may be employed herein.

Treatment of the polypeptide in the presence of one or more reducing agents such as tris(2-carboxyethyl) phosphine (TCEP) or dithiothreitol (DTT), optionally in the presence of urea, ammonium bicarbonate or any mixture thereof, in the appropriate conditions of temperature and time may be applicable. This step allows for disulphide reduction. The free sulfhydryl groups on the cysteine residues may subsequently be alkylated with reagents such as iodoacetamide (IAA) or iodoacetic acid to irreversibly prevent the free sulfhydryls from reforming disulphide bonds.

In preferred embodiments, a recombinant polypeptide according to the invention is reduced with DTT and alkylated with iodoacetamide during the purification steps.

In some embodiments, said polypeptide has a length ranging from 10 to 200 amino acid residues.

"From 10 to 200 amino acid residues" encompasses polypeptides having 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198 and 199 amino acid residues.

### • N-terminal domain of an α-gliadin

In some embodiments, the polypeptide according to the invention comprises the N-terminal domain of an α-gliadin, or a fragment of the N-terminal domain of an α-gliadin.

In some embodiments, the "N-terminal domain of an α-gliadin" does not comprise any cysteine residue.

In some embodiments, the "N-terminal domain of an α-gliadin" consists of the peptide region starting from the first amino acid residue at the N-terminus position, including at most the 1^{st} cysteine residue of the linear amino acid residues chain, and with the proviso that the N-terminal domain further includes no more than 7 amino acid residues consecutive to the said 1^{st} cysteine residue.

In some embodiments, a polypeptide comprising the N-terminal domain of an α-gliadin, or a fragment of the N-terminal domain of an α-gliadin comprises a plurality of peptides, said peptides being recognized by IgEs from gluten allergic individuals, and said peptides being selected in a group of peptides having at least 70% amino acid identity with peptides represented by the sequences SEQ ID No. 9 to SEQ ID No. 33 and by the sequences SEQ ID No. 65 to SEQ ID No. 77.

A skilled the art may understand that said peptides from a plurality of peptides are linearly adjacent to one another, or spatially distant from one another and therefore separated by a stretch of amino acids.

Within the scope of the present invention, the expression "a plurality of peptides" is intended to encompass at least 2, at least 3, at least 4, at least 5, at least 6, at least 7, at least 8 peptides.

Within the scope of the present invention, the expression "at least 2 peptides" encompasses 2, 3, 4, 5, 6, 7, 8, 9, 10 or more peptides; the expression "at least 3 peptides" encompasses 3, 4, 5, 6, 7, 8, 9, 10 or more peptides; the expression "at least 4 peptides" encompasses 4, 5, 6, 7, 8, 9, 10 or more peptides; the expression "at least 5 peptides" encompasses 5, 6, 7, 8, 9, 10 or more peptides; the expression "at least 6 peptides" encompasses 6, 7, 8, 9, 10 or more peptides; the expression "at least 7 peptides" encompasses 7, 8, 9, 10 or more peptides; the expression "at least 8 peptides" encompasses 8, 9, 10 or more peptides.

In some embodiments, a polypeptide comprising the N-terminal domain of an α-gliadin, or a fragment of the N-terminal domain of an α-gliadin comprises a plurality of peptides, said peptides being recognized by IgEs from gluten allergic individuals, and said peptides being selected in a group of peptides represented by the sequences SEQ ID No. 9 to SEQ ID No. 33 and by the sequences SEQ ID No. 65 to SEQ ID No. 77.

In some preferred embodiments, a polypeptide comprising the N-terminal domain of an α-gliadin, or a fragment of the N-terminal domain of an α-gliadin comprises a plurality of peptides, said peptides being recognized by IgEs from gluten allergic individuals, and said peptides being selected in a group of peptides having at least 70% amino acid identity with peptides represented by the sequences SEQ ID No. 9, SEQ ID No. 11, SEQ ID No. 12, SEQ ID No. 14, SEQ ID No. 16, SEQ ID No. 17, SEQ ID No. 18, SEQ ID No. 19, SEQ ID No. 20, SEQ ID No. 22, SEQ ID No. 23, SEQ ID No. 24, SEQ ID No. 27, SEQ ID No. 29, SEQ ID No. 31 and SEQ ID No. 33.

In some preferred embodiments, a polypeptide comprising the N-terminal domain of an α-gliadin, or a fragment of the N-terminal domain of an α-gliadin comprises a plurality of peptides, said peptides being recognized by IgEs from gluten allergic individuals, and said peptides being selected in a group of peptides represented by the sequences SEQ ID No. 9, SEQ ID No. 11, SEQ ID No. 12, SEQ ID No. 14, SEQ ID No. 16, SEQ ID No. 17, SEQ ID No. 18, SEQ ID No. 19, SEQ ID No. 20, SEQ ID No. 22, SEQ ID No. 23, SEQ ID No. 24, SEQ ID No. 27, SEQ ID No. 29, SEQ ID No. 31 and SEQ ID No. 33.

In some further preferred embodiments, a polypeptide comprising the N-terminal domain of an α-gliadin, or a fragment of the N-terminal domain of an α-gliadin comprises a plurality of peptides, said peptides being recognized by IgEs from gluten allergic individuals, and said peptides being selected in a group of peptides having at least 70% amino acid identity with peptides represented by the sequences SEQ ID No. 9, SEQ ID No. 12, SEQ ID No. 14 and SEQ ID No. 17.

In some further preferred embodiments, a polypeptide comprising the N-terminal domain of an α-gliadin, or a fragment of the N-terminal domain of an α-gliadin comprises a plurality of peptides, said peptides being recognized by IgEs from gluten allergic individuals, and said peptides being selected in a group of peptides represented by the sequences SEQ ID No. 9, SEQ ID No. 12, SEQ ID No. 14 and SEQ ID No. 17.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 9, SEQ ID No. 10 and SEQ ID No. 11.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 9, SEQ ID No. 10 and SEQ ID No. 11.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 14 and SEQ ID No. 16.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 14 and SEQ ID No. 16.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 13 and SEQ ID No. 15.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 13 and SEQ ID No. 15.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 9, SEQ ID No. 11, SEQ ID No. 18, SEQ ID No. 19 and SEQ ID No. 20.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 9, SEQ ID No. 11, SEQ ID No. 18, SEQ ID No. 19 and SEQ ID No. 20.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 22, SEQ ID No. 23, SEQ ID No. 24 and SEQ ID No. 27.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 22, SEQ ID No. 23, SEQ ID No. 24 and SEQ ID No. 27.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 21, SEQ ID No. 25 and SEQ ID No. 26.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 21, SEQ ID No. 25 and SEQ ID No. 26.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 29 and SEQ ID No. 30.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 29 and SEQ ID No. 30.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 28 and SEQ ID No. 30.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 28 and SEQ ID No. 30.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 9, SEQ ID No. 31 and SEQ ID No. 33.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 9, SEQ ID No. 31 and SEQ ID No. 33.

In some preferred embodiments, a polypeptide comprising the N-terminal domain of an α-gliadin, or a fragment of the N-terminal domain of an α-gliadin comprises the peptides recognized by IgEs from gluten allergic individuals and having at least 70% amino acid identity with peptides represented by the sequences SEQ ID No. 9, SEQ ID No. 31 and SEQ ID No. 32.

In some preferred embodiments, a polypeptide comprising the N-terminal domain of an α-gliadin, or a fragment of the N-terminal domain of an α-gliadin comprises the peptides recognized by IgEs from gluten allergic individuals and represented by the sequences SEQ ID No. 9, SEQ ID No. 31 and SEQ ID No. 32.

In some embodiments, the polypeptide comprising the N-terminal domain of an α-gliadin, or a fragment of the N-terminal domain of an α-gliadin has at least 70% amino acid identity with the polypeptide of SEQ ID No. 5.

In some embodiments, the polypeptide comprising the N-terminal domain of an α-gliadin, or a fragment of the N-terminal domain of an α-gliadin consists of the polypeptide of SEQ ID No. 5.

### • N-terminal domain of a γ-gliadin

In some embodiments, the polypeptide according to the invention comprises the N-terminal domain of a γ-gliadin, or a fragment of the N-terminal domain of a γ-gliadin.

In certain embodiments, the "N-terminal domain of a γ-gliadin" encompasses the region of amino acids residues belonging to the N-terminal domain of a γ-gliadin preceding and not including the 1^{st} cysteine residue.

In other words, the "N-terminal domain of a γ-gliadin" may not comprise any cysteine residue.

In some embodiments, the "N-terminal domain of a γ-gliadin" consists of the peptide region comprising the 1^{st} cysteine residue, with the proviso that the N-terminal domain further includes no more than 7.

In some embodiments, a polypeptide comprising the N-terminal domain of a γ-gliadin, or a fragment of the N-terminal domain of a γ-gliadin comprises a plurality of peptides, said peptides being recognized by IgEs from gluten allergic individuals, and said peptides being selected in a group of peptides having at least 70% amino acid identity with peptides represented by the sequences SEQ ID No. 34 to SEQ ID No. 64 and by the sequences SEQ ID No. 78 to SEQ ID No. 83.

In some embodiments, a polypeptide comprising the N-terminal domain of a γ-gliadin, or a fragment of the N-terminal domain of a γ-gliadin comprises a plurality of peptides, said peptides being recognized by IgEs from gluten allergic individuals, and said peptides being selected in a group of peptides represented by the sequences SEQ ID No. 34 to SEQ ID No. 64 and by the sequences SEQ ID No. 78 to SEQ ID No. 83.

In some embodiments, the polypeptide comprising the N-terminal domain of a γ-gliadin, or a fragment of the N-terminal domain of a γ-gliadin comprises a plurality of peptides, said peptides being recognized by IgEs from gluten allergic individuals, and said peptides being selected in a group of peptides having at least 70% amino acid identity with peptides represented by the sequences SEQ ID No. 34, SEQ ID No. 35, SEQ ID No. 37, SEQ ID No. 38, SEQ ID No. 39, SEQ ID No. 40, SEQ ID No. 41, SEQ ID No. 42, SEQ ID No. 43, SEQ ID No. 45, SEQ ID No. 47, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 59, SEQ ID No. 60, SEQ ID No. 62, SEQ ID No. 63 and SEQ ID No. 64.

In some embodiments, the polypeptide comprising the N-terminal domain of a γ-gliadin, or a fragment of the N-terminal domain of a γ-gliadin comprises a plurality of peptides, said peptides being recognized by IgEs from gluten allergic individuals, said peptides being selected in a group of peptides represented by the sequences SEQ ID No. 34, SEQ ID No. 35, SEQ ID No. 37, SEQ ID No. 38, SEQ ID No. 39, SEQ ID No. 40, SEQ ID No. 41, SEQ ID No. 42, SEQ ID No. 43, SEQ ID No. 45, SEQ ID No. 47, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 59, SEQ ID No. 60, SEQ ID No. 62, SEQ ID No. 63 and SEQ ID No. 64.

In some embodiments, the polypeptide comprising the N-terminal domain of a γ-gliadin, or a fragment of the N-terminal domain of a γ-gliadin comprises a plurality of peptides, said peptides being recognized by IgEs from gluten allergic individuals, said peptides being selected in a group of peptides having at least 70% amino acid identity with peptides represented by the sequences SEQ ID No. 35, SEQ ID No. 43, SEQ ID No. 50, SEQ ID No. 57, SEQ ID No. 60 and SEQ ID No. 64.

In some embodiments, the polypeptide comprising the N-terminal domain of a γ-gliadin, or a fragment of the N-terminal domain of a γ-gliadin comprises a plurality of peptides, said peptides being recognized by IgEs from gluten allergic individuals, said peptides being selected in a group of peptides represented by the sequences SEQ ID No. 35, SEQ ID No. 43, SEQ ID No. 50, SEQ ID No. 57, SEQ ID No. 60 and SEQ ID No. 64.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 34, SEQ ID No. 37, SEQ ID No. 38, SEQ ID No. 39, SEQ ID No. 40 and SEQ ID No. 41.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 34, SEQ ID No. 37, SEQ ID No. 38, SEQ ID No. 39, SEQ ID No. 40 and SEQ ID No. 41.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 34, SEQ ID No. 36 and SEQ ID No. 41.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 34, SEQ ID No. 36 and SEQ ID No. 41.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 42 and SEQ ID No. 45.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 42 and SEQ ID No. 45.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 42 and SEQ ID No. 44.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 42 and SEQ ID No. 44.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 47, SEQ ID No. 49, SEQ ID No. 51, SEQ ID No. 52, SEQ ID No. 53, SEQ ID No. 54 and SEQ ID No. 55.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 47, SEQ ID No. 49, SEQ ID No. 51, SEQ ID No. 52, SEQ ID No. 53, SEQ ID No. 54 and SEQ ID No. 55.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 46 and SEQ ID No. 48.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 46 and SEQ ID No. 48.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 56 and SEQ ID No. 57.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 56 and SEQ ID No. 57.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 56 and SEQ ID No. 58.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 56 and SEQ ID No. 58.

In some embodiments, the polypeptide comprising the N-terminal domain of a γ-gliadin, or a fragment of the N-terminal domain of a γ-gliadin comprises the peptides recognized by IgEs from gluten allergic individuals and having at least 70% amino acid identity with peptides represented by the sequences SEQ ID No. 62, SEQ ID No. 63 and SEQ ID No. 64.

In some embodiments, the polypeptide comprising the N-terminal domain of a γ-gliadin, or a fragment of the N-terminal domain of a γ-gliadin comprises the peptides recognized by IgEs from gluten allergic individuals and represented by the sequences SEQ ID No. 62, SEQ ID No. 63 and SEQ ID No. 64.

In some embodiments, the polypeptide comprising the N-terminal domain of a γ-gliadin, or a fragment of the N-terminal domain of a γ-gliadin comprises the peptide recognized by IgEs from gluten allergic individuals and having at least 70% amino acid identity with peptides represented by the sequence SEQ ID No. 61.

In some embodiments, the polypeptide comprising the N-terminal domain of a γ-gliadin, or a fragment of the N-terminal domain of a γ-gliadin comprises the peptide recognized by IgEs from gluten allergic individuals and represented by the sequence SEQ ID No. 61.

In some embodiments, the polypeptide comprising the N-terminal domain of a γ-gliadin, or a fragment of the N-terminal domain of a γ-gliadin has at least 70% amino acid identity with the polypeptide of SEQ ID No. 6.

In some embodiments, the polypeptide comprising the N-terminal domain of a γ-gliadin, or a fragment of the N-terminal domain of a γ-gliadin consists of the polypeptide of SEQ ID No. 6.

### • Hetero-polypeptide

In some embodiments, a polypeptide according to the invention is a hetero-polypeptide comprising, in a one single amino acids sequence,
- at least one N-terminal domain of an α-gliadin, or a fragment of the N-terminal domain of an α-gliadin, and
- at least one N-terminal domain of a γ-gliadin, or a fragment of the N-terminal domain of a γ-gliadin. Any combination of specific peptides described above may be generated to provide such a hetero-polypeptide.

In some embodiments, a hetero-polypeptide comprising, in a one single amino acids sequence,
- at least one N-terminal domain of an α-gliadin, or a fragment of the N-terminal domain of an α-gliadin, and
- at least one N-terminal domain of a γ-gliadin, or a fragment of the N-terminal domain of a γ-gliadin,
may be for use as an agent for desensitization of an individual with an IgE-dependent gluten allergy, resulting from a cross-allergy between α-gliadin allergy and γ-gliadin allergy.

In some embodiments, said hetero-polypeptide has a length ranging from 10 to 400 amino acid residues.

"From 10 to 400 amino acid residues" encompasses hetero-polypeptides having 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390 and 395 amino acid residues.

In some embodiments, the hetero-polypeptide according to the invention may comprise:
- at least one peptide recognized by IgEs from gluten allergic individuals, said peptide being selected in a group of peptides having at least 70% amino acid identity with peptides represented by the sequences SEQ ID No. 9 to SEQ ID No. 33; and
- at least one peptide recognized by IgEs from gluten allergic individuals, said peptide being selected in a group of peptides having at least 70% amino acid identity with peptides represented by the sequences SEQ ID No. 34 to SEQ ID No. 64.

### Immunogenic composition

Another aspect of the invention relates to an immunogenic composition comprising:
- a polypeptide or a mixture of polypeptides as defined in the instant invention ; and
- at least one physiologically acceptable adjuvant.

Within the scope of the invention, the expression "immunogenic composition" encompasses a composition able to elicit an immunogenic response after being in contact with a living animal or human body. By "immunogenic response", a skilled in the art understands a response feature by the production of IgG antibodies, in a human body.

Within the scope of the invention, the expression "a mixture of polypeptides" encompasses at least 2, at least 3, at least 4, at least 5, at least 6, at least 7, at least 8, at least 9, at least 10 polypeptides according to the invention.

In some embodiments, said mixture may comprise polypeptides comprising the N-terminal domain of α-gliadins or γ-gliadins originating from the same plant species, or a fragment thereof. In some embodiments, said mixture may comprise polypeptides comprising the N-terminal domain of α-gliadins or γ-gliadins originating from different plant species, or a fragment thereof.

In some embodiments, said mixture may comprise polypeptides comprising the N-terminal domain of α-gliadin(s) and γ-gliadin(s) originating from the same plant species, or a fragment thereof. In some embodiments, said mixture may comprise polypeptides comprising the N-terminal domain of α-gliadin(s) and γ-gliadin(s) originating from different plant species, or a fragment thereof.

Physiologically acceptable adjuvants encompass, but are not limited to, Stimulon™, QS-21 (Aquila Biopharmaceuticals, Inc., Framingham, Mass.); MPL™ (3-O-deacylated monophosphoryl lipid A; Corixa, Hamilton, Mont.), 529 (an amino alkyl glucosamine phosphate compound, Corixa, Hamilton, Mont.), IL-12 (Genetics Institute, Cambridge, Mass.); GM-CSF (Immunex Corp., Seattle, Wash.); N-acetyl-muramyl-L-theronyl-D-isoglutamine (thr-MDP); N-acetyl-nor-muramyl-L-alanyl-D-isoglutamine (CGP 11637, referred to as nor-MDP); N-acetylmuramyl-L-alanyl-D-isoglutaminyl-L-alanine-2-(1'-2'-dipalmitoyl-sn-glycero-3-hydroxyphosphoryloxy-ethylamine) (CGP 19835A, referred to as MTP-PE); and cholera toxin. Other physiologically acceptable adjuvants that may be used encompass non-toxic derivatives of cholera toxin, including its A subunit, and/or conjugates or genetically engineered fusions of the *N. meningitidis* polypeptide with cholera toxin or its B subunit ("CTB"), procholeragenoid, fungal polysaccharides, including schizophyllan, muramyl dipeptide, muramyl dipeptide ("MDP") derivatives, phorbol esters, the heat labile toxin of *E. coli,* block polymers or saponins.

In certain embodiments, the composition according to the invention claim 13, wherein the said polypeptide(s) is(are) covalently linked to a carrier molecule.

In certain embodiments, a polypeptide according to the invention is advantageously covalently linked to a carrier molecule when said polypeptide has a length comprised between 4 and 29 amino acids.

The types of carrier molecules routinely used for generating an immunogenic product comprising a polypeptide according to the invention linked to a carrier molecule are belonging to the common knowledge of a skilled in the art. The carrier molecule is intended to provide cytokine help (or T-cell help) in order to enhance the immune response against the allergenic polypeptide according to the invention.

The carrier molecule to which the peptide is optionally bound can be selected from a wide variety of known carriers. Examples of carrier molecules for immunotherapy purposes encompass proteins such as human or bovine serum albumin and keyhole limpet haemocyanin (KLH) and fatty acids. Other embodiments of carrier molecules to which an antigenic polypeptide according to the invention may be covalently linked include bacterial toxins or toxoids, such as diphtheria, cholera, *E. coli* heat labile or tetanus toxoids, the *N. meninigitidis* outer membrane protein (European patent application n° EP0372501), synthetic peptides (European patent applications n° EP0378881 and n° EP0427347), heat shock proteins (PCT application n° WO93/17712), *Pertussis* proteins (PCT application n° WO98/58668), protein D from *H. influenzae* (PCT application n° WO00/56360.) and toxin A or B from *C. difficile* (International patent application WO00/61761).

Any suitable conjugation reaction can be used, with any suitable linker where necessary.

In some embodiments, the immunogenic composition according to the invention is in the form of a live attenuated vaccine comprising a recombinant live organism, of viral or bacterial origin, the said live organism expressing one or more polypeptide(s) as defined above.

Within the scope of the present invention, the expression "live attenuated vaccine" encompasses vaccine compositions containing a version of a living microbe that has been modified so that it cannot elicit the corresponding disease when administered in a living animal or human body. Live attenuated vaccine may elicit strong cellular and antibody responses and often confer lifelong immunity with only one or two doses.

In some embodiments, a recombinant live organism of viral origin may be selected in a group comprising the vaccine virus, the poliomyelitis virus, adenoviruses and retroviruses.

In some embodiments, a recombinant live organism of bacterial origin may be selected in a group comprising *Salmonella typhi* strains, *Mycobacterium bovis* strains, *Listeria monocytogenes* strains, *Bordetella pertussis* strains, *Shigella sp.* strains, *Streptococcus gordonii* strains, *Lactococcus lactis* strains, *Lactobacillus sp.* strains, *Staphylococcus xylosus* strains and *Staphylococcus carnosus* strains.

Within the scope of the present invention, said recombinant live organism is expressing one or more polypeptide(s) as defined above. In practice, recombinant DNA encoding said polypeptide(s) are incorporated in said virus or said bacteria, directly in the genome of said live organism.

In some embodiments, bacterial live attenuated organism may include a plasmid or cosmid vector comprising a DNA encoding said polypeptide(s) to be produced.

Genetic engineering techniques to provide such recombinant live organisms, notably the techniques allowing for DNA recombination, DNA cloning, DNA incorporation into bacteria, selection of the appropriate promoters are within the skills of an artisan in the art.

In some embodiments, the immunogenic composition according to the invention is for use as a medicament.

In some embodiments, the immunogenic composition according to the invention is for use as an antigenic compound.

In some embodiments, the immunogenic composition according to the invention is for use as an agent for desensitization of an IgE-dependent gluten allergy.

The formulation of such immunogenic compositions is well known to persons skilled in the art. Immunogenic compositions of the invention preferably include at least one pharmaceutically acceptable excipient. Suitable pharmaceutically acceptable excipients include, but are not limited to, any and all conventional solvents, dispersion media, fillers, solid carriers, aqueous solutions, coatings, antibacterial and antifungal agents, isotonic and absorption delaying agents, and the like. Suitable pharmaceutically acceptable excipients include, for example, water, saline buffer, phosphate buffered saline buffer, dextrose, glycerol, ethanol and the like, as well as combinations thereof. Pharmaceutically acceptable excipients may further comprise minor amounts of auxiliary substances such as wetting or emulsifying agents, preservatives, which enhance the shelf life or effectiveness of the immunogenic composition. The preparation and use of pharmaceutically acceptable excipients is well known in the art. Except insofar as any conventional media or agent is incompatible with the polypeptide according to the invention, use thereof in the immunogenic compositions of the present invention is contemplated.

Such immunogenic compositions can be administered parenterally, e.g., by injection, either subcutaneously or intramuscularly, as well as orally or intranasally. Other modes of administration employ oral formulations, pulmonary formulations, suppositories, and transdermal applications, for example, without limitation. Oral formulations, for example, include such normally employed excipients as, for example, pharmaceutical grades of mannitol, lactose, starch, magnesium stearate, sodium saccharine, cellulose, magnesium carbonate, and the like, without limitation.

### Desensitization method

The present invention also relates to a method for desensitization of an individual with an IgE-dependent gluten allergy comprising a step of administering to said individual an immunogenic composition comprising a polypeptide comprising a N-terminal domain of an α-gliadin or a γ-gliadin, or a fragment thereof, optionally linked to a carrier molecule.

In certain embodiments, an immunogenic composition according to the invention comprises a polypeptide comprising a N-terminal domain of an α-gliadin or a γ-gliadin, or a fragment thereof, in an amount which is suitable for the administration of from 1 µg to 1000 mg, which encompasses from 10 µg to 1 mg, of said polypeptide to an individual in need thereof, for therapeutic purposes.

The amount of said polypeptide to be administered to an individual in need thereof depends of the age, the gender, the weight and the further medical condition of said individual and may be of at least 200 µg, 500µg, or 1mg, preferably at most 1000 mg.

The amount of said polypeptide specified above also applies when the immunogenic composition comprises said polypeptide in a conjugated form with a carrier molecule.

In certain embodiments, an immunogenic composition is administered at least twice to an individual in need thereof. In these embodiments, the second step of administration of an immunogenic composition according to the invention is performed in a time period of from 2 weeks to 6 months after the first administration step.

In certain embodiments, an immunogenic composition is administered at least three times to an individual in need thereof. In these embodiments, the second step of administration of the said immunogenic composition according to the invention is performed in a time period of from 2 weeks to 6 months after the first administration step. In these embodiments, the third step of administration of the said immunogenic composition is performed in a time period of from more than 6 months to about one year after the first administration step.

In some embodiments, the said immunogenic composition is then again administered to a desensitized individual, for example every 5-year time period or every 10-year time period.

### EXAMPLE

The present example aims to characterize the structural features linked to allergenicity of proteins belonging to the prolamin superfamily by focusing on two members: α- and γ-gliadins. Different forms (natural/recombinant; native/reduced-alkylated) and fragments (Nt- and Ct-) of α- and γ-gliadins were produced to evaluate the contribution of their structural domains and disulphide bonds to IgE binding. Their secondary structure was determined using synchrotron radiation circular dichroism (SRCD); sera from patients with food allergies to wheat (FAW) or baker's asthma (BA) were used to analyse IgE binding by ELISA and Pepscan; and biological activity was measured by monitoring rat basophil elicitation.

### 1 MATERIALS AND METHODS

### 1.1 PCR cloning of whole gliadins cDNA and of their moieties.

rAG (SEQ ID No. 3) and rNt-AG (SEQ ID No. 5) have been previously cloned from wheat (*Triticum aestivum*) (Mameri et al.; J. Agric. Food Chem; 2012, 60, 8059-8068). The same procedure was used for cloning recombinant γ-gliadin and other fragments. Briefly, total RNA was extracted from immature wheat grains (*Triticum aestivum*). α-gliadin (rAG; SEQ ID No. 3) and γ-gliadin (rGG; SEQ ID No. 4) cDNA were cloned by RT-PCR using Superscript II™ system (Invitrogen). Their Nt- and Ct- moieties, namely rNt-AG (SEQ ID No. 5), rAG-Ct (SEQ ID No. 7), rNt-GG (SEQ ID No. 6) and rGG-Ct (SEQ ID No. 8) were cloned by PCR. The PCR was run directly using cDNA as template. The DNA Polymerase used was AccuPrime™ Pfx (Life Technologies). Fragments were cloned with restriction enzymes into a pET28b (Novagen) plasmid in frame with a sequence encoding the hexa-histidine Tag (His-Tag). The sequences of the specific primers used in the cloning procedure of rAG (SEQ ID No. 3) and rNt-AG (SEQ ID No. 5) are described in Mameri et al. (see above).

The sequences of the specific primers used in the cloning of rAG-Ct (SEQ ID No. 7), rGG (SEQ ID No. 4), rNt-GG (SEQ ID No. 6) and rGG-Ct (SEQ ID No. 8) are indicated in the Table 1 below:

**Table 1**

| | | |
|---|---|---|
| primer 1 for cloning of rAG-Ct | CACGGATCCATGCAGGGATGTCGTCTTG | SEQ ID No. 84 |
| primer 2 for cloning of rAG-Ct | CTCGAGTTATGAGTTAGTACCGAAGATGCCA | SEQ ID No. 85 |
| primer 1 for cloning of rGG ¹ | CGATGGCAACAACTATCG | SEQ ID No. 86 |
| primer 2 for cloning of rGG ¹ | TCATTGTCCACTGATGCC | SEQ ID No. 87 |
| primer 3 for cloning of rGG ¹ | CACCATGAATATGCAGGTCGACCCTAG | SEQ ID No. 88 |
| primer 1 for cloning of rGG-Ct | ACTCCATGGCTTGCAAGAATTACCTCT | SEQ ID No. 89 |
| primer 2 for cloning of rGG-Ct | GTGCTCGAGTCCACTGATGCCGGCGACTAT | SEQ ID No. 90 |
| primer 1 for cloning of rNt-GG | CACCATGAATATGCAGGTCGACCCTAG | SEQ ID No. 91 |
| primer 2 for | TTGTAGATATGGCTGAATCAACG | SEQ ID No. 92 |
| cloning of rNt-GG | | |

| | | |
|---|---|---|
| Nested PCR technique is used, the 1^{st} PCR is performed with primers 1 and 2; a 2^{nd} PCR is subsequently performed using the PCR product from the 1^{st} PCR, with primers 1 and 3. | | |

### 1.2 Production and purification of natural or recombinant gliadins and fragments

Natural α- and γ-gliadins, nAG (SEQ ID No. 1) and nGG (SEQ ID No. 2), were prepared by several steps of chromatography (Battais et al.; J. Cereal. Sci.; 2005, 42, 109-117). The γ-gliadin corresponded to the y46 fraction described in (Egorov et al.; Biochem; 1998, 63, 1061-1067). When indicated, natural proteins were used after reduction/alkylation. After solubilisation in 0.5 M Tris-HCl, pH 8.5 containing 8 M urea and 0.01M EDTA, they were reduced with 0.1 M dithiothreitol (DTT) for 2 h at 37°C, and then alkylated with iodoacetamide in 0.5 M NaOH for 30 min in the dark. *E. coli* strain Rosetta™ (Novagen) was used for recombinant protein production. Bacteria were transformed with a plasmid encoding rAG (SEQ ID No. 3) or rGG (SEQ ID No. 3) or their respective Nt- or Ct-domains. Protein production and purification were performed as described in (Mameri et al. J. Agric. Food Chem; 2012, 60, 7538-7547) with standard culture and IPTG induction, lysis with lysozyme in the presence of RNase A and Dnase I (Qiagen) and precipitation steps. Proteins were reduced with DTT and alkylated with iodoacetamide during purification to increase extraction yields except when native full-length proteins were produced. The purity of materials was assessed by SDS-PAGE on a 15% w/w gel; 5 µg of proteins were loaded on the gel and the proteins were stained with Coomassie Brilliant Blue R250. The presence of Nt-histidines in recombinant proteins was confirmed by western blot using an anti-HisTag antibody.

### 1.3 Natural α-gliadin identification by mass spectrometry

nAG (SEQ ID No. 1; 3 mg/ml) was hydrolysed at 37°C for 6 h using porcine trypsin (Sigma T8253) and Chymotrypsin (Sigma C 4879) in 25 mM NH₄HCO₃ at an enzyme/substrate ratio of 1/100 w/w. The resulting hydrolysate was reduced by mixing with 10 mM DTT and 10% acetonitrile at 45°C for 1 h in the dark with gentle stirring. After cooling the sample to room temperature, 0.5 M iodoacetamide was added and the sample was incubated for 30 min in the dark. Following reduction/alkylation, the peptides were diluted ten times in water; subsequently, 50 µl of the solution was eluted on C18 resin pipet tips (ZipTip, Millipore Corp, Billerica, MA), cleaned and finally extracted from the Zip Tip with a 30 µl of 1:1 acetonitrile/water containing 0.05% formic acid and 50% acetonitrile. The acetonitrile was then evaporated on speed vac and the sample was stored for mass spectrometry analysis.

Nanoscale capillary liquid chromatography-tandem mass spectrometry (LC-MS/MS) analyses were performed using an Ultimate U3000 RSLC system (Dionex) coupled with an LTQ-Orbitrap VELOS mass spectrometer (Thermo Fisher). Chromatographic separation and mass data acquisition were performed as described in Lupi et al. (J. Cereal Sc.; 2014, 60, 179-186). Protein identification was performed using the Mascot database search engine and the UniProt databank, which was restricted to *Poaceae* sequences (release 2015_01). Mass tolerance for peptide precursors was set at 5 ppm, with the fragment mass tolerance set at 0.5 Da. Enzyme specificity was set to trypsin and chymotrypsin with a maximum of 1 mis-cleavage.

### 1.4 Synchrotron radiation circular dichroism (SRCD)

Measurements were taken using the DISCO beam line at Soleil synchrotron (Gif-sur-Yvette, France). The samples were prepared in 50 mM sodium phosphate buffer pH 8 containing 2% SDS and 10 mM sodium sulphate (typical range 0.5-1.5 g/l). Data acquisition and processing were recorded as previously described (Vindigni et al. BBA Biomembr. 2013, 1828, 1881-1888). Ratios of secondary structures were determined using the ContinLL program (van Stockkum etal. Anal. Biochem. 1990, 191, 110-118) in Dichroweb (Whitmore et al. Nucl. Ac. Res. 2004, 32, W668-W673) and SP175 or Set5 as reference sets on the mean spectra from 5 measurements. Normalized root-mean-square deviation (NRMSD) indicated the most accurate fit for each spectrum, values less than 0.25 were considered as significant.

### 1.5 Sera from allergic patients

Sera from 25 patients with food allergy to wheat (FAW) and from 5 patients with baker's asthma (BA) were selected based on the presence of gliadin, nAG (SEQ ID No. 1) or nGG (SEQ ID No. 2), specific IgE as determined by ELISA. All patients with BA were adults and presented rhinitis and asthma. Expert for one adult, all patients with FAW were children. Their symptoms included atopic eczema dermatitis syndrome, anaphylaxis, asthma, urticaria or gastro-intestinal disorders. Sensitization to wheat proteins was assessed in patients using skin prick tests (SPT) to wheat flour (Moulins Soufflet), gluten (ALK-Abello) and to natural gliadins and/or using specific-IgE assays to wheat flour or gluten (Phadia ImmunoCAP). A wheat allergy was confirmed by a positive challenge or evident effect of wheat avoidance. The double-blind placebo-controlled food challenge and the inhalation challenge with wheat flour are described in Bensefa et al. (Allergy, 2004, 59, 833-838) and Denery-Papini et al. (Clin. Exp. Allergy, 2011, 41, 1478-1492), respectively. Blood collection, SPT and challenges were performed with the informed consent of the patients or their parents and after receiving approval from the biomedical research by the Ethics Committee of Ile de France III and AFSSAPS (authorization number 2008-A01565-50).

### 1.6 IgE binding to gliadins and their domains

Specific IgE concentrations toward the different forms of AG and GG (natural/recombinant; native/reduced-alkylated) and their Nt- or Ct-domains were determined by F-ELISA as described in Mameri et al. (Molecul. Nutr. Food Res., 2012, 56, 1847-1883) using alkaline-phosphatase-conjugated goat anti-human IgE and 4-MUP as a substrate (Sigma A3525 and A3168, respectively, Saint-Quentin Fallavier, France). Proteins solubilized in PBS were coated at 5 mg/l in carbonate buffer on white 384-well plates (NUNC 460372, Fischer Scientific).

### 1.7 IgE binding to solid-phase synthetic peptides (Pepscan)

Sera containing high levels of specific-IgE toward the gliadins and their domains (n=6 for AG and, n=5 for GG) and 3 control sera from non-atopic subjects were used in the Pepscan analysis as described in Mameri et al. (Molecul. Nutr. Food Res., 2012, 56, 1847-1883). Deca-peptides, overlapping on 8 amino acid residues, spanned the full-length sequences of α and γ-gliadins (UniProt ID P04725 and P08453, respectively), which were already used in previous Pescan analysis (Denery-Papini et al. Clin. Exp. Allergy, 2011, 41, 1478-1492; Battais et al. Allergy 2005, 60, 815-821).

### 1.8 Activation ofRBL cells by α-gliadins and their domains

Rat basophil leukaemia (RBL SX38) cell degranulation was expressed as the percentage of β-hexosaminidase release with a positive threshold set at 11% as described in Mameri et al. (Molecul. Nutr. Food Res. 2012, 56, 1874-1883). Gliadin samples were tested at 2, 20, 200 and 2000 ng/ml with a pool of 2 sera (899+907) diluted 1/10.

### 1.9 Statistical analysis

Statistical analyses were performed using GraphPad Prism version 5.04 for Windows (GraphPad software, San Diego, CA). For the ELISA results, paired tests were performed and each pairing was confirmed to be significantly effective. Both specific IgE distributions and their paired differences failed the d'Agostino-Pearson omnibus normality test. Wilcoxon signed rank tests or Friedman tests with Dunn's multiple comparisons were then performed to compare the medians. P-values less than 0.05 were considered significant.

### 2 RESULTS

### 2.1 Characterization of natural and recombinant gliadins and their domains

The full-length sequences of the recombinant and natural gliadins are shown in Table 1. The recombinant α-gliadin (rAG; SEQ ID No. 3) sequence shares 96% identity with the protein that was recorded under UniProt ID P02863.2. MS analysis of the natural α-gliadin (nAG; SEQ ID No. 1) indicated that it contained in majority the α-gliadin ID Q3S4V7, in addition to several minor isoforms of this protein. Sequence alignment revealed 90% identity between rAG (SEQ ID No. 3) and nAG (SEQ ID No. 1). In SDS-PAGE under reducing conditions, rAG (SEQ ID No. 3) migrated to approximately 45 kDa, whereas the nAG (SEQ ID No. 1) migrated to approximately 40 kDa (Figure1). The recombinant γ-gliadin (rGG; SEQ ID No. 4) was recorded under UniProt ID Q94G94. The purified natural γ-gliadin corresponded to a γ46-gliadin type according to its electrophoretic mobility in Acid-PAGE and was previously described as protein ID P08453. The rGG (SEQ ID No. 4) and nGG (SEQ ID No. 2) sequences displayed 72 % identity. Their mobility in SDS-PAGE was approximately 40 kDa. The Nt- and Ct- recombinant domains each constitute approximately one half of a gliadin protein and migrated approximately 15-22 kDa. Despite their equal sizes, the mobility of the rNt-GG fragment (133 residues; SEQ ID No. 6) was faster than that of the rGG-Ct fragment (136 residues; SEQ ID No. 8). The molecular weights of the prolamins are often overestimated by SDS-PAGE, which has been attributed to the high content of proline in their Nt- repetitive domains.

### 2.2 Secondary structures of natural and recombinant gliadins and their domains

The secondary structure contents of gliadins and of their domains were calculated after the deconvolution of the CD spectra. Low NRMSD values were obtained except for the rNt- GG (SEQ ID No. 6) domain (0.27).

The CD spectra of α-gliadins presented minima values in the 206-208 and 220-224 nm regions, which reflect the α-helical structures. nAG (SEQ ID No. 1) contained approximately 35% α-helix, 18% turns and a low level of β-strand. Reduction/alkylation of the protein did not impact the dichroic signal (Figure 2A). rAG (SEQ ID No. 3) and its domains displayed similar dichroic signals (Figure 2C) corresponding to a higher level of α-helix (52-58 %) and a lower level of turn structures (11%) compared with nAG (SEQ ID No. 1).

The secondary structure contents of nGG (SEQ ID No. 2) were approximately 25% α-helix and 16% β-strand and turns, and the reduction/alkylation of nGG (SEQ ID No. 2) did not change the CD spectrum (Figure 2B).

The recombinant rGG (SEQ ID No. 4) contained slightly more α-helix (37%) and fewer strands (11%) than did nGG (SEQ ID No. 2). Unlike rAG (SEQ ID No. 3), full-length rGG (SEQ ID No. 4) and its Nt-domain (SEQ ID No. 6) and Ct-domain (SEQ ID No. 8) displayed different spectra (Figure 2D). rGG-Ct domain (SEQ ID No. 8) is notably folded in α-helices (67%). The CD spectrum of the repetitive rNt-GG domain (SEQ ID No. 6) showed an unusual negative peak at 210 nm, which is characteristic of turn structures. This spectrum could only be poorly deconvoluted with the currently available sets (NRMSD higher than 0.25); hence, its secondary structure was not evaluated.

### 2.3 Serum identification and clinical characteristics of patients allergic to wheat

Sera from 25 patients with food allergy to wheat (top) and 5 patients with baker's asthma (bottom) were used. Patients' reactivity was tested by Skin Prick Test, CAP® Phadia and ELISA and food/respiratory challenge. AEDS: atopic eczema dermatitis syndrome; AS: anaphylactic shock; Asth: Asthma; U: urticaria; GI: gastrointestinal symptoms; nd: not determined; nAG: natural α-gliadins, nGG: natural γ-gliadins. Results of food /respiratory challenges: pos: positive.

**Table 2 below indicates the sera's characteristics of patients allergic to wheat**

| | | | | **Skin Prick Test (mm)** | | | | **Specific IgE in CAP® KUI/L** | | **Specific IgE in ELISA ng/ml** | | **Food or respiratory Challenge** |
|---|---|---|---|---|---|---|---|---|---|---|---|---|
| **Sera** | **Sex/age** | | **symptoms** | **wheat** | **Gluten** | **nAG** | **nGG** | **wheat** | **gluten** | **nAG** | **nGG** | |
| **121** | F | 3 | AEDS | 5 | 4 | nd | nd | > 100 | 0 | 104 | 37 | Pos 5 g flour |
| **130** | M | 2 | AS | 7 | 4 | nd | nd | > 100 | 20 | 243 | 158 | Pos 1 g flour |
| **134** | M | 8 | AEDS | pos | nd | nd | nd | > 100 | nd | 59 | 0 | Pos 1 g bread |
| **269** | F | 2 | AEDS | 2 | 2 | nd | nd | nd | nd | 59 | 106 | Pos 13 g flour |
| **292** | F | 4 | U | 3 | 3 | nd | nd | 16.4 | 4.1 | 38 | 49 | Pos 5 g flour |
| **447** | F | 9 | AEDS, Asth | 5 | 1.5 | 5 | 0 | 12.5 | 12.6 | 38 | 11 | Pos 1,3 g bread |
| **450** | M | 15 | GI | 4 | 3 | 5,5 | 0 | 13.1 | 6.3 | 37 | 8 | Pos 10 g flour |
| **451** | M | 4 | AEDS | 15.5 | 10.5 | 14 | 0 | >10 0 | >10 0 | 283 | 3.2 | nd |
| **590** | F | 8 | AS | nd | nd | nd | nd | nd | nd | 119 | 107 | nd |
| **653** | M | 5 | GI | 0 | 0 | 0 | 0 | 0 | 0 | 0 | 17 | Pos 21 g flour |
| **669** | M | 8 | GI | 6 | 3 | nd | nd | 4.8 | Nd | 24 | 14 | nd |
| **695** | F | 6 | AEDS, Asth | 15 | 6 | 6.5 | 0 | >100 | >100 | 450 | 344 | Pos 2 g bread |
| **728** | M | 8 | U, Asth | 6 | nd | nd | nd | nd | 36 | 221 | 38 | Pos 2 g flour |
| **779** | M | 3 | AEDS, Asth | 7 | 8.5 | 10 | 0 | 93.3 | 95.5 | 262 | 55 | Pos 6 g bread |
| **885** | M | 52 | U, Asth | 3 | 3 | 0 | 0 | 2.42 | 3.74 | 45 | 41 | nd |
| **895** | F | 15 | AS | 10 | 15 | nd | nd | nd | 8.6 | 45 | 1 | nd |
| **899** | M | 5 | U, Asth | 5 | 6 | nd | nd | >10 0 | >10 0 | 388 | 252 | nd |
| **901** | M | 5 | U, GI | 5 | 5 | nd | nd | > 100 | > 100 | 409 | 104 | nd |
| **907** | M | 10 | AS | nd | nd | nd | nd | > 100 | > 100 | 472 | 139 | Pos 1 g flour |
| **998** | F | 2 | AEDS | 3 | nd | nd | nd | 86 | nd | 23 | 0 | nd |
| **100 0** | M | 12 | GI | 8 | 4 | 7 | 5 | nd | nd | 228 | 177 | nd |
| **100 2** | M | 9 | AS | 6 | 7 | nd | nd | 60.2 | 73.2 | 159 | 44 | nd |
| **102 6** | M | 11 | AEDS, Asth | 7.5 | 3.5 | 9 | 0 | 38 | 23 | 202 | 1 | Pos |
| **106 8** | M | 11 | AS | 5 | 6 | nd | nd | 10.1 | 7.96 | 79 | 35 | Pos 2 g flour |
| **106 9** | M | 2 | U | 10 | 10 | nd | nd | 10 | 10 | 40 | 13 | nd |
| **630** | F | 55 | rhinitis, Asth | 2 | 0 | nd | nd | nd | nd | 16 | 1 | Pos |
| **855** | M | 56 | rhinitis, Asth | 5,5 | 0 | 0 | 6 | 44 | nd | 13 | 1 | Pos |
| **864** | M | 30 | rhinitis, Asth | nd | nd | nd | nd | nd | nd | 36 | 0 | nd |
| **107 6** | M | 23 | rhinitis, Asth | nd | nd | nd | nd | 16 | nd | 47 | 0 | nd |
| **108 9** | M | 51 | rhinitis, Asth + AS with cofactor | 10 | 0 | 0 | 0 | 26 | 16.2 | 13 | 0 | nd |

### 2.4 IgE binding to natural and recombinant gliadins and the involvement of disulphide bonds

Sera from 28 patients with FAW or BA were used to compare IgE-reactivity toward different forms of α-gliadins in an ELISA. As shown in Figure 3A, compared with nAG (SEQ ID No. 1), the IgE binding to rAG (SEQ ID No. 3) was significantly reduced. The mean of the specific IgE concentration was reduced by 60%, but 25 of the 28 sera retained binding to rAG (SEQ ID No. 3). Reduction/alkylation highly decreased the IgE binding to nAG (SEQ ID No. 1): the mean reduction was approximately 70%, and 67% of the sera exhibited a reduction of more than 70%. Reduction/alkylation had a lower but significant impact on IgE binding to rAG (SEQ ID No. 3), with a 30 % reduction. Mean and median values are indicated in Table 3 below.

**Table 3**

| | **Specific IgE (ng/ml)** | | | |
|---|---|---|---|---|
| | **nAG** (SEQ ID No. 1) | **R/A nAG** (SEQ ID No. 1) | **rAG** (SEQ ID No. 3) | **R/A rAG** (SEQ ID No. 3) |
| Mean | 147 | 50 | 62 | 43 |
| Median | 69 | 19 | 20 | 16 |

Sera from 20 patients with FAW were used to compare IgE reactivity toward γ-gliadins. The sera from the 5 patients with BA had no or very weak IgE reactivity toward nGG (SEQ ID No. 2). As shown in Figure 3B, compared with nGG (SEQ ID No. 2), IgE binding to rGG (SEQ ID No. 4) was reduced. The reduction in the mean specific IgE concentration was approximately 30%, and 18 of the 20 sera retained IgE binding after replacing nGG (SEQ ID No. 2) with rGG (SEQ ID No. 4). The decrease in the mean specific IgE concentration induced by the reduction/alkylation of nGG (SEQ ID No. 2) was approximately 60%, and 37% of the sera exhibited a reduction over 70%. In case of rGG (SEQ ID No. 4), a significant decrease of approximately 60 % was also observed. Mean and median values are indicated in Table 4 below.

**Table 4**

| | **Specific IgE (ng/ml)** | | | |
|---|---|---|---|---|
| | **nGG (SEQ ID No. 2)** | **R/A nGG** (SEQ ID No. 2) | **rGG** (SEQ ID No. 4) | **R/A rGG** (SEQ ID No. 4) |
| Mean | 88 | 37 | 60 | 26 |
| Median | 47 | 26 | 34 | 20 |

### 2.5 Involvement of Nt- and Ct- domains in IgE binding to gliadins

The involvement of the two structural domains of gliadins in IgE-binding was analysed with recombinant repetitive domains (rNt) and non-repetitive domains (rCt). rCt domains were extracted after the reduction/alkylation of cysteines. Because it was previously shown that reduction/alkylation could impact IgE binding to gliadins, comparisons were performed with reduced/alkylated full-length recombinant gliadins, and only sera with positive IgE binding to R/A recombinant gliadins were considered (Figure 4). The IgE-binding data were analysed by a Friedman test, which indicated that the rAG (SEQ ID No. 3) and rGG (SEQ ID No. 4) full-length and fragments (p<0.0001; F=19.4, n=21 for AG and F=26.8, n=17 for GG) were significantly different. For both gliadins, a subsequent Dunn's comparison test comparing the domains with the full-length protein revealed that the differences for the Ct-domain (difference in rank sum=28.0 for AG, 20.0 for GG) were significant but that the differences were not significant (p>0.05) for the Nt-domain. Mean and median values are indicated in Table 5 below.

**Table 5**

| | **Specific IgE (ng/ml)** | | | | | |
|---|---|---|---|---|---|---|
| | **R/A rAG** (SEQ ID No.3) | **rNt-AG** (SEQ ID No.5) | **rAG-Ct** (SEQ ID No.7) | **R/A rGG** (SEQ ID No.4) | **rNt-GG** (SEQ ID No.6) | **rGG-Ct** (SEQ ID No.8) |
| Mean | 57 | 39 | 20 | 32 | 36 | 6 |
| Median | 22 | 19 | 5 | 26 | 24 | 0 |

The mean specific IgE concentration was twice as high for the rNt-AG (SEQ ID No. 5) as it was for rAG-Ct (SEQ ID No. 7) (39 and 20 ng/mL, respectively); IgE from 43% of sera did not bind the Ct-domain, whereas it did bind the Nt-domain. The sum of the mean specific IgE concentrations for the two domains was equivalent to the mean concentration for the full-length recombinant gliadin after reduction/alkylation (57 ng/mL). The rGG-Ct domain (SEQ ID No. 8) was rarely bound by specific IgE (only 25% of the sera). Conversely, all sera displayed IgE binding to the rNt-GG (SEQ ID No. 6). The mean concentrations of specific IgE for rNt-GG and R/A rGG binding were equivalent (36 and 32 ng/mL).

The involvement of the two structural gliadin domains in IgE binding was also analysed by searching for continuous peptide epitopes within these sequences. As shown in Tables 6 and 7 below, IgE from all 6 tested sera bound sequences belonging to the Nt-domain of α-gliadin, whereas two sera (n° 451 and 590) did not bind any sequence located in the Ct-domain. Stretches of peptides were detected with several sera: the sequences VRVPVPQLQP (SEQ ID No. 9), VQQQQFPG, QPYLQLQPFPQQPFPPQLP and QSFPPQQPYPQQ (SEQ ID No. 29) were found in the Nt-domain.

**Table 6: IgE reactivity of sera from wheat allergic patients toward synthetic peptides screened by Pepscan analysis.**

| **serum** | n | **Nt-AG** sequences |
|---|---|---|
| **130** | 10 | 1VRVPVPQLQP10 25VQQQFPGQQQFPPQQP42 53QPYLQLQPFPQPQPFPPQLP72 |
| **451** | 14 | 23PLVQQQQFPGQQQQ36 |
| | | 41QPYPQPQPFPSQQPYLQLQPFPQPQPFPPQLPYPQPQS78 |
| **590** | 23 | ¹VRVPVPQLQP¹⁰ ²⁵VQQQFPGQQQFPPQQPYPQPQPFPSQQP⁵⁴ ⁵³QPYLQLQPFPQPQPFPPQLP⁷²    ⁷⁷QSFPPQQPYPQQQP⁹⁰ ⁹⁵PQQPISQQQAQQ¹⁰⁶ |
| **899** | 35 | ¹VRVPVPQLQPQNPSQQQPQEQVPLVQQQQFPGQQQQFPPQQ PYPQPQPFPSQQPYLQLQPFPQPQPFPPQLPYPQPQS⁷⁸ ⁷⁷QSFPPQQPYPQQQP⁹⁰ ⁸⁷QQQPQYLQPQQPISQQQAQQ¹⁰⁶ |
| **901** | 5 | **⁷³YPQPQSFPPQQPYPQQ**^{**88** 113}QQQQQQQILQ¹²² |
| **1068** | 7 | ¹VRVPVPQLQP¹⁰ ²³PLVQQQQFPG³² ⁶⁵FPPQLPYPQPQSFPPQQPYPQQQP⁸⁹ |

Numbers of spots (n) detected in Nt-domain of AG (Uniprot accession number P04725) are indicated together with stretches of sequences in which spots are detected with low (normal) and high (bold) intensity. Peptides detected with the highest intensity by serum are underlined.

The five sera tested on the γ-gliadin sequence displayed IgE binding to 7 to 38 peptides located in the Nt-domain (see Table 7). The numerous peptides in the Nt-domain corresponding to the consensus sequences PQQPFPQQPQ, LSQQPQQTFP (SEQ ID No. 35), QPQQSFPQ or QPQQTFPQ were found with all sera, reflecting a higher homogeneity of repetition in γ-gliadin than in α-gliadin. There is no relationship between the number of peptides detected by Pepscan analysis and the IgE binding measured by ELISA toward the respective domains.

**Table 7: IgE reactivity of sera from wheat allergic patients toward synthetic peptides screened by Pepscan analysis.**

| **serum** | n | **Nt-GG** sequences |
|---|---|---|
| **130** | 32 | **²⁵LSQQPQQTFPQQPQQTFPHQP⁴⁴** |
| | | ⁵¹PQQPQQPFLQPQFPQQPQQPFPQTQQPQQPFPQQPQQPFPQTQ QPQQPFPQQPQQPFPQTQQPQQPFPQLQQPQQPFPQPQQ¹³⁴ |
| | | **¹³⁹PQQPQQSFPQQQ¹⁵⁰** |
| **590** | 26 | ⁵⁷PFLQPQQPFPQQPQQPFPQTQQPQQPFPQQPQQPFPQTQQPQ QPFPQQPQQPFPQTQQPQQPFPQLQQPQQPFPQPQQ¹³⁴ ¹⁴¹QPQQSFPQQQRP¹⁵² |
| **899** | 38 | 25LSQQPQQTFPQPQQTFPHQPQQQVPQPQ52 53QPQQPFLQPQQPFPQQPQQPFPQTQQPQQPFPQQPQQPFPQTQQ QQPQQPFPQQPQQPFPQTQQPQQPFPQLQQPQQPFPQPQQQLPQ PQQPQQSFPQQQRP152 |
| **901** | 7 | **²⁵LSQQPQQTFPQPQQTFPHQP^{43 137}QPQQPQQSFPQQQ¹⁵⁰** |
| **1068** | 16 | ⁵⁹LQPQQPFPQQPQQPFPQTQQPQQPFPQQPQQPFPQTQQPQQPFP QQPQQPFPQTQQPQQPFPQLQQPQQPFPQPQQ¹³⁴ |

Numbers of spots (n) detected in Nt-domain of GG (Uniprot accession number P08453) are indicated together with stretches of sequences in which spots are detected with low (normal) and high (bold) intensity. Peptides detected with the highest intensity by serum are underlined.

Table 8 below indicates the different peptides identified by PEPSCAN analysis in selected serum for the Nt-domain of α-gliadin (SEQ ID No. 9 to SEQ ID No. 33) and the Nt-domain of γ-gliadin (SEQ ID No. 34 to SEQ ID No. 64).

### 2.6 Triggering potential of natural, recombinant and Nt- and Ct-fragments of α-gliadin

The triggering potential of α-gliadin samples was assessed in an RBL assay using a pool of 2 sera that displayed high levels of specific IgE toward nAG (SEQ ID No. 1; approximately 400 ng/mL), rAG (SEQ ID No. 3; approximately 250 ng/mL), R/A nAG (SEQ ID No. 1; approximately 200 ng/mL), R/A rAG (SEQ ID No. 3; approximately 150 ng/mL), and the gliadin domains (70 ng/mL for rNt-AG (SEQ ID No. 5) and of 100 ng/mL for rAG-Ct (SEQ ID No. 7).

Both nAG (SEQ ID No. 1) and rAG (SEQ ID No. 3) were able to induce cell degranulation in the presence of the pool of sera (Figure 5). However, maximum degranulation (approximately 35%) was obtained at a lower protein concentration for nAG (SEQ ID No. 1; 20 ng/mL) than for rAG (SEQ ID No. 3; 200 ng/mL). Reduced/alkylated nAG (SEQ ID No. 1) or rAG (SEQ ID No. 3) induced low degranulation at similar concentration (200 ng/ml), and none of the recombinant domains were able to induce degranulation above the positive threshold.

### 3 CONCLUSION

Wheat gliadins are frequently involved in FAW and occasionally involved in BA.

Like all wheat storage proteins, α- and γ-gliadins are polymorphic and have closely related sequences. The recombinant gliadins rAG (SEQ ID No. 3) and rGG (SEQ ID No. 4) displayed 90 and 72% sequence identity to the isoforms present in the natural AG (SEQ ID No. 1) and GG (SEQ ID No. 2) purified fractions, respectively. These recombinant proteins displayed higher contents in α-helical secondary structure (52% for rAG (SEQ ID No. 3) and 36% for rGG (SEQ ID No. 4)) compared with the natural fractions (36% for nAG (SEQ ID No. 1) and 25% for nGG (SEQ ID No. 2)). The results from SRCD are in accordance with the previously published data.

Recombinant gliadins showed reduced IgE binding compared with natural gliadins, but 90% of the sera were still reactive. We also observed that rAG (SEQ ID No. 3) retained biological activity.

The disruption of disulphide bonds in AG and GG led to reduced IgE binding to these allergens for many sera. This disruption also reduced the degranulation potency of AG. Disulphide bonds are located in the Ct-domain of both proteins. Protein reduction likely disrupted some of the conformational epitopes. Only one serum (n°901) bound to a cysteine-containing peptide of AG by Pepscan analysis. However, reduction may have an indirect but drastic effect on the global folding of the protein. When the proteins were reduced/alkylated, no significant effect was observed on the secondary structures of natural AG and GG. Reduction/alkylation likely modified the tertiary structures of these proteins, but unfortunately, all attempts to crystallize them failed. These results indicate that gliadins with their disulphide bonds are more IgE-reactive for many sera than are gliadins without disulphide bonds. The importance of disulphide bonds for IgE binding has already been shown for other allergens of the prolamin superfamily.

IgE binding to peptides and recombinant domains of AG indicated the presence of epitopic regions in both domains of this protein. Nevertheless, stronger binding to the Nt-domain compared with the Ct-domain was observed both by the Pepscan technique and using the recombinant domains. The secondary structure contents of the Nt- and Ct-domains were equivalent, with the high level of α-helix most likely due to the clusters of 13 to 15 glutamines present in both domains. Recombinant rNt-AG polypeptide (SEQ ID No. 5) and synthetic peptides may adopt a fold representing, to a certain extent, the natural Nt-domain, allowing IgE to cross-react with these different entities. Conversely, IgE cross-reactions between the natural Ct-domain and the corresponding (reduced) recombinant domain and synthetic peptides should be less likely. In the case of GG, this phenomenon was more pronounced because very low binding to the Ct-domain was observed by both Pepscan and ELISA using the recombinant domain, whereas numerous continuous epitopes were detected in the Nt-domain.

The CD spectra clearly indicated that the two domains have different secondary structures: the rNt-GG (SEQ ID No. 6) primarily contains turn structures, whereas the rGG-Ct (SEQ ID No. 8) contains a very high content of α-helix. Despite sequences homology and similarities in their organization, differences were observed in patients' reactivity toward α- and γ-gliadins: allergenicity of GG was less affected by the reduction of disulphide bridges and more pronounced towards its Nt-domain than in the case of AG.

Both domains and correctly formed disulphide bonds in the Ct-domain appeared to be required for AG to induce basophile degranulation. This result differs from the reported presence of continuous repetitive IgE epitopes in deamidated ω2-gliadins or fragments from the repetitive domain of HMW glutenin that could induce basophile degranulation (Baar et al. Allergy, 2014, 69, 1316-1323; Denery-Papini, et al. Allergy, 2012, 67, 1023-1032.).

Using recombinant domains of gliadins is a complementary approach to synthetic peptides and expands the possibilities of epitope research by accounting, to a certain extent, for protein folding. The proteins in their native form should be preferentially used for IgE reactivity detection. Disulphide bonds in the Ct-domain most likely play a role in generating conformational epitopes within this domain or are formed by a combination of residues from both domains. Indeed, studies with Nt- and Ct- fragments of LMW glutenin subunits demonstrated interactions between both parts and their importance in IgE binding (Mameri et al. J. Agric. Food Chem. 2012, 60, 7538-7547).

The availability of IgE-reactive proteins becomes very important in understanding the mechanisms of wheat allergies and provides new approaches in diagnosis or therapy.

**Table 8: Peptides sequences used herein**

| SEQ ID No. | | Observation |
|---|---|---|
| 1 | VRVPVPQLQPQNPSQQQPQEQVPLVQQQFLGQ QQPFPPQQPYPQPQPFPSQQPYLQLQPFPQPQLPY PQPQPFRPQQPYPQPQPQYSQPQQPISQQQQQQ QQQQQQQILQQILQQQLIPCMDVVLQQHNIVH GRSQVLQQSTYQLLRELCCQHLWQIPEQSQCQA IHNVVHAIILHQQQKQQQQPSSQVSFQQPLQQYP LGQGSFRPSQQNPQAQGSVQPQQLPQFEEIRNLA LQTLPAMCNVYIPPYCTIAPFGIFGTN | Natural α-gliadin from *Triticum aestivum* (nAG) |
| 2 | NIQVDPSGQVQWLQQQLVPQLQQPLSQQPQQTF PQPQQTFPHQPQQVPQPQQPQQPFLQPQQPFPQ QPQQPFPQTQQPQQPFPQQPQQPFPQTQQPQQPF PQQPQQPFPQTQQPQQPFPQLQQPQQPFPQPQQQ LPQPQQPQQSFPQQQRPFIQPSLQQQLNPCKNILL QQCKPASLVSSLWSIIWPQSDCQVMRQQCCQQL AQIPQQLQCAAIHSVVHSIIMQQQQQQQQGID IFLPLSQHEQVGQGSLVQGQGIIQPQQPAQLEAIR SLVLQTLPSMCNVYVPPECSIMRAPFASIVAGIG GQ | Natural γ-gliadin from *Triticum aestivum* (nGG) |
| 3 | GSSHHHHHHSQDPVRVPMPQLQPQNPSQQQPQE QVPLVQQQFPGQQQPFPPQQPYPQPQPFPSQQP YLQLQPFPQPQLPYPQPQPFRPQQPYPQPQPQYS QPQQPISQQQQQQQQQQQQQILQQILQQQL IPCRDVVLQQHNIAHASSQVLQQSSYQLLQQLC CQRLWQIPEQSRCQAIHNVVHAIILQQQQQQQ QQQQPSSQVSYQQPQQQYPSGQGSFQPSQQNSQ | Recombinant α-gliadin from *Triticum aestivum* (rAG) |
| | AQGSVQSQQLPQFEEIRNLVLQTLPAMCNVYIPP YCSTTIAPFGIFGTNS | |
| 4 | NMQVDPSGQVQWPQQQPFRQPQQPFYQQPQQT FPQPQQTFPHQPQQQFPQPQQPQQFPQPQQPQQ PFPQPQQAQLPFPQQPQQPFPQPQQPQQPFPQSQ QPQQPFPQPQQPQQSFPQQQQPLIQPYLQQQMNP CKNYLLQQCNPVSLVSSLVSMILPRSDCQVMQQ QCCQQLAQIPRQLQCAAIHSVVHSIIMQQEQQQG IQILRPLFQLVQGQGIIQPQQPAQYEVIRSLVLRT LPNMCNVYVRPDCSTINAPFASIVAGISGQLEHH HHHH | Recombinant γ-gliadin from *Triticum aestivum* (rGG) |
| 5 | GSSHHHHHHSQDPVRVPMPQLQPQNPSQQQPQE QVPLVQQQFPGQQQPFPPQQPYPQPQPFPSQQP YLQLQPFPQPQLPYPQPQPFRPQQPYPQPQPQYS QPQQPISQQQQQQQQQQQQQILQQILQQQL IPCRDVVLQQ | Recombinant N-ter domain of α-gliadin from *Triticum aestivum* (rNt-AG) |
| 6 | NMQVDPSGQVQWPQQQPFRQPQQPFYQQPQQT FPQPQQTFPHQPQQQFPQPQQPQQFPQPQQPQQ PFPQPQQAQLPFPQQPQQPFPQPQQPQQPFPQSQ QPQQPFPQPQQPQQSFPQQQQPLIQPYLQQQMNP | Recombinant N-ter domain of γ-gliadin from *Triticum aestivum* (rNt-GG) |
| 7 | QLIPCRDVVLQQHNIAHASSQVLQQSSYQLLQQ LCCQRLWQIPEQSRCQAIHNVVHAIILQQQQQ QQQQQQPSSQVSYQQPQQQYPSGQGSFQPSQQN SQAQGSVQSQQLPQFEEIRNLVLQTLPAMCNVYI PPYCSTTIAPFGIFGTNS | Recombinant C-ter domain of α-gliadin from *Triticum aestivum* (rAG-Ct) |
| 8 | QMNPCKNYLLQQCNPVSLVSSLVSMILPRSDCQ VMQQQCCQQLAQIPRQLQCAAIHSVVHSIIMQQ EQQQGIQILRPLFQLVQGQGIIQPQQPAQYEVIRS LVLRTLPNMCNVYVRPDCSTINAPFASIVAGISG QLEHHHHHH | Recombinant C-ter domain of γ-gliadin from *Triticum aestivum* (rGG-Ct) |
| 9 | VRVPVPQLQP | Peptide from N-ter domain of α-gliadin (sera n° 130; 590; 1068) |
| 10 | VQQQQFPGQQQQFPPQQP | Peptide from N-ter domain of α-gliadin (serum n° 130) |
| 11 | QPYLQLQPFPQPQPFPPQLP | Peptide from N-ter domain of α-gliadin (sera n° 130; 590) |
| 12 | YLQLQPFPQP | Peptide from N-ter domain of α-gliadin (serum n° 130) |
| 13 | PLVQQQQFPGQQQQ | Peptide from N-ter domain of α-gliadin (serum n° 451) |
| 14 | QQQFPGQQQQ | Peptide from N-ter domain of α-gliadin (serum n° 451) |
| 15 | QPYPQPQPFPSQQPYLQLQPFPQPQPFPPQLPYPQ PQS | Peptide from N-ter domain of α-gliadin (serum n° 451) |
| 16 | QPYPQPQPFPSQQPYLQLQPFPQPQPFPPQLPYPQ P | Peptide from N-ter domain of α-gliadin (serum n° 451) |
| 17 | QPQPFPSQQP | Peptide from N-ter domain of α-gliadin (serum n° 451) |
| 18 | VQQQQFPGQQQQFPPQQPYPQPQPFPSQQP | Peptide from N-ter domain of α-gliadin (serum n° 590) |
| 19 | QSFPPQQPYPQQQP | Peptide from N-ter domain of α-gliadin (serum n° 590) |
| 20 | PQQPISQQQAQQ | Peptide from N-ter domain of α-gliadin (serum n° 590) |
| 21 | VRVPVPQLQPQNPSQQQPQEQVPLVQQQQFPGQ QQQFPPQQPYPQPQPFPSQQPYLQLQPFPQPQPFP PQLPYPQPQS | Peptide from N-ter domain of α-gliadin (serum n° 899) |
| 22 | VRVPVPQLQPQN | Peptide from N-ter domain of α-gliadin (serum n° 899) |
| 23 | PLVQQQQFPGQQQQFPPQQP | Peptide from N-ter domain of α-gliadin (serum n° 899) |
| 24 | QPYLQLQPFPQP | Peptide from N-ter domain of α-gliadin (serum n° 899) |
| 25 | QSFPPQQPYPQQQP | Peptide from N-ter domain of α-gliadin (serum n° 899) |
| 26 | QQQPQYLQPQQPISQQQAQQ | Peptide from N-ter domain of α-gliadin (serum n° 899) |
| 27 | QPISQQQAQQ | Peptide from N-ter domain of α-gliadin (serum n° 899) |
| 28 | YPQPQSFPPQQPYPQQ | Peptide from N-ter domain of α-gliadin (serum n° 901) |
| 29 | QSFPPQQPYPQQ | Peptide from N-ter domain of α-gliadin (serum n° 901) |
| 30 | QQQQQQQILQ | Peptide from N-ter domain of α-gliadin (serum n° 901) |
| 31 | PLVQQQQFPG | Peptide from N-ter domain of α-gliadin (serum n° 1068) |
| 32 | FPPQLPYPQPQSFPPQQPYPQQQP | Peptide from N-ter domain of α-gliadin (serum n° 1068) |
| 33 | FPPQLPYPQPQSFPPQQPYPQQ | Peptide from N-ter domain of α-gliadin (serum n° 1068) |
| 34 | LSQQPQQTFPQPQQTFPHQP | Peptide from N-ter domain of γ-gliadin (serum n° 130) |
| 35 | LSQQPQQTFP | Peptide from N-ter domain of γ-gliadin (serum n° 130) |
| 36 | PQQPQQPFLQPQFPQQPQQPFPQTQQPQQPFPQQ PQQPFPQTQQPQQPFPQQPQQPFPQTQQPQQPFP QLQQPQQPFPQPQQ | Peptide from N-ter domain of γ-gliadin (serum n° 130) |
| 37 | PQQPQQPFLQPQFPQQPQQPFPQT | Peptide from N-ter domain of γ-gliadin (serum n° 130) |
| 38 | PQQPFPQQ | Peptide from N-ter domain of γ-gliadin (serum n° 130) |
| 39 | QPFPQTQQPQQPFPQQPQQPFPQT | Peptide from N-ter domain of γ-gliadin (serum n° 130) |
| 40 | PQQPFPQLQQPQQPFPQPQQ | Peptide from N-ter domain of γ-gliadin (serum n° 130) |
| 41 | PQQPQQSFPQQQ | Peptide from N-ter domain of γ-gliadin (serum n° 130) |
| 42 | PFLQPQQPFPQQPQQPFPQTQQPQQPFPQQPQQP FPQTQQPQQPFPQQPQQPFPQTQQPQQPFPQLQQ PQQPFPQPQQ | Peptide from N-ter domain of γ-gliadin (serum n° 590) |
| 43 | LQPQQPFPQQ | Peptide from N-ter domain of γ-gliadin (serum n° 590) |
| 44 | QPQQSFPQQQRP | Peptide from N-ter domain of γ-gliadin (serum n° 590) |
| 45 | QPQQSFPQQQ | Peptide from N-ter domain of γ-gliadin (serum n° 590) |
| 46 | LSQQPQQTFPQPQQTFPHQPQQQVPQPQ | Peptide from N-ter domain of γ-gliadin (serum n° 899) |
| 47 | LSQQPQQTFPQPQQTFPHQPQQQVPQ | Peptide from N-ter domain of γ-gliadin (serum n° 899) |
| 48 | QPQQPFLQPQQPFPQQPQQPFPQTQQPQQPFPQQ PQQPFPQTQQQPQQPFPQQPQQPFPQTQQPQQP FPQLQQPQQPFPQPQQQLPQPQQPQQSFPQQQRP | Peptide from N-ter domain of γ-gliadin (serum n° 899) |
| 49 | LQPQQPFPQQPQ | Peptide from N-ter domain of γ-gliadin (serum n° 899) |
| 50 | LQPQQPFPQQ | Peptide from N-ter domain of γ-gliadin (serum n° 899) |
| 51 | QTQQ | Peptide from N-ter domain of γ-gliadin (serum n° 899) |
| 52 | QQPQ | Peptide from N-ter domain of γ-gliadin (serum n° 899) |
| 53 | FPQTQQQQPQQPFPQQPQ | Peptide from N-ter domain of γ-gliadin (serum n° 899) |
| 54 | FPQTQQPQQPFPQLQQPQQPFPQPQQ | Peptide from N-ter domain of γ-gliadin (serum n° 899) |
| 55 | QPQQSFPQQQ | Peptide from N-ter domain of γ-gliadin (serum n° 899) |
| 56 | LSQQPQQTFPQPQQTFPHQP | Peptide from N-ter domain of γ-gliadin (serum n° 901) |
| 57 | QQPQQTFPQP | Peptide from N-ter domain of γ-gliadin (serum n° 901) |
| 58 | PQPQQPQQSFPQQQ | Peptide from N-ter domain of γ-gliadin (serum n° 901) |
| 59 | PQQPQQSFPQQQ | Peptide from N-ter domain of γ-gliadin (serum n° 901) |
| 60 | QPQQSFPQQQ | Peptide from N-ter domain of γ-gliadin (serum n° 901) |
| 61 | LQPQQPFPQQPQQPFPQTQQPQQPFPQQPQQPFP QTQQPQQPFPQQPQQPFPQTQQPQQPFPQLQQPQ QPFPQPQQ | Peptide from N-ter domain of γ-gliadin (serum n° 1068) |
| 62 | LQPQQPFPQQPQ | Peptide from N-ter domain of γ-gliadin (serum n° 1068) |
| 63 | QQPQQPFPQQPQ | Peptide from N-ter domain of γ-gliadin (serum n° 1068) |
| 64 | QQPQQPFPQPQQ | Peptide from N-ter domain of γ-gliadin (serum n° 1068) |
| 65 | QEQVPLVQQQ | Peptide from N-ter domain of α-gliadin |
| 66 | VQQQQFPGQQ | Peptide from N-ter domain of α-gliadin |
| 67 | QFPGQQQQFP | Peptide from N-ter domain of α-gliadin |
| 68 | PGQQQQFPPQ | Peptide from N-ter domain of α-gliadin |
| 69 | QQQQFPPQQP | Peptide from N-ter domain of α-gliadin |
| 70 | PQQPYPQPQP | Peptide from N-ter domain of α-gliadin |
| 71 | QPFPSQQPYL | Peptide from N-ter domain of α-gliadin |
| 72 | FPSQQPYLQL | Peptide from N-ter domain of α-gliadin |
| 73 | SQQPYLQLQP | Peptide from N-ter domain of α-gliadin |
| 74 | YPQQQPQYLQ | Peptide from N-ter domain of α-gliadin |
| 75 | QILQQILQQQ | Peptide from N-ter domain of α-gliadin |
| 76 | QILQQQLIPC | Peptide from N-ter domain of α-gliadin |
| 77 | QVPLVQQQQF | Peptide from N-ter domain of α-gliadin |
| 78 | LAQIPQQLQC | Peptide from N-ter domain of γ-gliadin |
| 79 | QQQLPQPQQP | Peptide from N-ter domain of γ-gliadin |
| 80 | LQQQLVPQLQ | Peptide from N-ter domain of γ-gliadin |
| 81 | QQLVPQLQQP | Peptide from N-ter domain of γ-gliadin |
| 82 | QQSFPQQQRP | Peptide from N-ter domain of γ-gliadin |
| 83 | QTQQPQQPFP | Peptide from N-ter domain of γ-gliadin |

### REFERENCES

Baar, A., Pahr, S., Constantin, C., Scheiblhofer, S., et al., Molecular and immunological characterization of Tri a 36, a low molecular weight glutenin, as a novel major wheat food allergen. J. Immunol. 2012, 189, 3018-3025.
Baar, A., Pahr, S., Constantin, C., Giavi, S., et al., The high molecular weight glutenin subunit Bx7 allergen from wheat contains repetitive IgE epitopes. Allergy 2014, 69, 1316-1323.
Battais, F., Pineau, F., Popineau, Y., Aparicio, C., et al., Food allergy to wheat: identification of immunogloglin E and immunoglobulin G-binding proteins with sequential extracts and purified proteins from wheat flour. Clin. Exp. Allergy 2003, 33, 962-970.
Battais, F., Courcoux, P., Popineau, Y., Kanny, G., et al., Food allergy to wheat : differences in IgE-Binding proteins as a function of age and symptoms. J. Cereal Sci. 2005, 42, 109-117.
Battais, F., Mothes, T., Moneret-Vautrin, D. A., Pineau, F., et al., Identification of IgE-binding epitopes on gliadins for patients with food allergy to wheat. Allergy 2005, 60, 815-821.
Bayer, E. and Mutter, M., Liquid Phase Synthesis of peptides. Nature 1972, 237,512 - 513.
Bensefa, L., Villette, C., Tabka, F., Causse-Sounillac, E., et al., Rye flour induces a stronger early bronchial response than wheat flour in occupational asthma. Allergy 2004, 59, 833-838.
Bittner, C., Grassau, B., Frenzel, K., Baur, X., Identification of wheat gliadins as an allergen family related to baker's asthma. J Allergy Clin Immunol 2008, 121, 744-749.
Camarca, A., Anderson, R. P., Mamone, G., Fierro, O., et al., Intestinal T Cell Responses to Gluten Peptides Are Largely Heterogeneous: Implications for a Peptide-Based Therapy in Celiac Disease. J. Immunol. 2009, 182, 4158-4166.
Denery-Papini, S., Bodinier, M., Pineau, F., Triballeau, S., et al., Immunoglobulin-E-binding epitopes of wheat allergens in patients with food allergy to wheat and in mice experimentally sensitized to wheat proteins. Clin. Exp. Allergy 2011, 41, 1478-1492.
Denery-Papini, S., Bodinier, M., Larré, C., Brossard, C., et al., Allergy to gluten isolates in patients tolerant to wheat: specific epitopes linked to deamidation. Allergy 2012, 67, 1023-1032.
Dupont, F. M., Vensel, W. H., Tanaka, C. K., Hurkman, W. J., Altenbach, S. B., Deciphering the complexities of the wheat flour proteome using quantitative two-dimensional electrophoresis, three proteases and tandem mass spectrometry. Proteome Sci. 2011,9, 10.
Egorov, T., Odintsova, T., Musolyamov, A., Barbashov, S., et al., Partial amino acid sequence of gamma-46 gliadin. Biochemistry 1998, 63, 1061-1067.
Green and Sambrook; Molecular Cloning, A Laboratory Manual; 4th Edition, 2012; Cold Spring Harbor Laboratory Press.
Hofmann, S. C., Fischer, J., Eriksson, C., Bengtsson Gref, O., et al., IgE detection to alpha/beta/gamma-gliadin and its clinical relevance in wheat-dependent exercise-induced anaphylaxis. Allergy 2012, 67, 1457-1460.
Huibregtse IL, Marietta EV, Rashtak S, Koning F, et al. Induction of antigen-specific tolerance by oral administration of Lactococcus lactis delivered immunodominant DQ8-restricted gliadin peptide in sensitized nonobese diabetic Abo Dq8 transgenic mice. J Immunol. 2009 Aug 15;183(4):2390-6.
Lupi, R., Masci, S., Rogniaux, H., Tranquet, O., et al., Assessment of the allergenicity of soluble fractions from GM and commercial genotypes of wheats. Journal of Cereal Science 2014, 60, 179-186.
Mäkelä, M. J., Eriksson, C., Kotaniemi-Syrjänen, A., Palosuo, K., et al., Wheat allergy in children - new tools for diagnostics. Clinical & Experimental Allergy 2014, 44, 1420-1430.
Mameri, H., Snégaroff, J., Gohon, Y., Pecquet, C., et al., Immunoglobulin-E Reactivity and Structural Analysis of Wheat Low-Molecular-Weight Glutenin Subunits and Their Repetitive and Nonrepetitive Halves. Journal of Agricultural and Food Chemistry 2012, 60, 7538-7547.
Mameri, H., Bouchez, I., Pecquet, C., Raison-Peyron, N., et al., A Recombinant ω-Gliadin-like D-Type Glutenin and an α-Gliadin from Wheat (Triticum aestivum): Two Immunoglobulin E Binding Proteins, Useful for the Diagnosis of Wheat-Dependent Allergies. Journal of Agricultural and Food Chemistry 2012, 60, 8059-8068.
Mameri, H., Denery-Papini, S., Pietri, M., Tranquet, O., et al., Molecular and immunological characterization of wheat Serpin (Tri a 33). Molecular Nutrition & Food Research 2012, 56, 1874-1883.
Matsuo, H., Kohno, K., Morita, E., Molecular cloning, recombinant expression and IgE-binding epitope of ω-5 gliadin, a major allergen in wheat-dependent exercise-induced anaphylaxis. FEBS J. 2005, 272, 4431-4438.
Maurano F, Siciliano RA, De Giulio B, Luongo D, et al. Intranasal administration of one alpha gliadin can downregulate the immune response to whole gliadin in mice. Scand J Immunol. 2001 Mar;53(3):290-5.
Merrifield, Solid Phase Peptide Synthesis. I. The Synthesis of a Tetrapeptide. J. Am. Chem. Soc. 85 (14): 2149-2154.
Metakovsky, E. V., Gliadin allele indentification in common wheat II. Catalogue of gliadin alleles in common wheat. J. Genet. Breed 1991, 45, 325-344.
Sander, I., Rozynek, P., Rihs, H.-P., van Kampen, V., et al., Multiple wheat flour allergens and cross-reactive carbohydrate determinants bind IgE in baker's asthma. Allergy 2011, 66, 1208-1215.
Senger S, Maurano F, Mazzeo MF, Gaita M, et al., Identification of immunodominant epitopes of alpha-gliadin in HLA-DQ8 transgenic mice following oral immunization. J Immunol. 2005 Dec 15;175(12):8087-95.
Tanabe, S., IgE-binding abilities of pentapeptides, QQPFP and PQQPF, in wheat gliadin. J. Nutr. Sci. Vitaminol. 2004, 50, 367-370.
Vindigni, J.-D., Wien, F., Giuliani, A., Erpapazoglou, Z., et al., Fold of an oleosin targeted to cellular oil bodies. Biochimica et Biophysica Acta (BBA) - Biomembranes 2013, 1828, 1881-1888.
Van Stokkum, I., Spoelder, H., Bloemendal, M., Van Grondelle, R., Groen, F., Estimation of protein secondary structure and error analysis from CD spectra. Anal. Biochem. 1990, 191, 110-118.
Waga, J., Obtulowicz, K., Zientarski, J. B., B., Czarnobilska, E., Stachowicz, M., Purified wheat gliadin proteins as immunoglobulin E binding factors in wheat mediated allergies. Am J Plant Sci 2011, 2, 476-483.
Whitmore, L., Wallace, B. A., DICHROWEB, an online server for protein secondary structure analyses from circular dichroism spectroscopic data. Nucleic Acids Res. 2004, 32, W668-W673.
Yokooji T, Kurihara S, Murakami T, Chinuki Y, et al. Characterization of causative allergens for wheat-dependent exercise-induced anaphylaxis sensitized with hydrolyzed wheat proteins in facial soap. Allergol Int. 2013 Dec;62(4):435-45.

## Claims

1. An immunogenic composition suitable for desensitization of an individual with diagnosed IgE-dependent gluten allergy comprising:
- a polypeptide or a mixture of polypeptides comprising the N-terminal domain of an α-gliadin, or a fragment of the N-terminal domain of an α-gliadin, wherein the polypeptide has at least 70% amino acid identity with the polypeptide of SEQ ID NO: 5 or with a polypeptide having a length ranging from 25 to 200 amino acid residues of SEQ ID NO: 5; and
- at least one physiologically acceptable adjuvant.

2. Immunogenic composition according to claim 1, wherein the said polypeptide is a recombinant polypeptide.

3. Immunogenic composition according to claim 1 or 2, wherein the said polypeptide is a reduced and/or alkylated polypeptide.

4. Immunogenic composition according to any one of claims 1 to 3, wherein the said polypeptide has a length of 25, 26, 27, 28, 29, 30, 31, 32, 33, 34, 35, 36, 37, 38, 39, 40, 41, 42, 43, 44, 45, 46, 47, 48, 49 or 50 amino acid residues.

5. Immunogenic composition according to any one of claims 1 to 4, wherein the said polypeptide has at least 95% amino acid identity with the polypeptide of SEQ ID No. 5 or with a polypeptide having a length ranging from 25 to 200 amino acid residues of SEQ ID NO: 5.

6. Immunogenic composition according to any one of claims 1 to 5, wherein the said polypeptide consists of the polypeptide of SEQ ID No. 5 or of a polypeptide having a length ranging from 25 to 200 amino acid residues of SEQ ID NO: 5.

7. Immunogenic composition according to any one of claims 1 to 6, wherein the said polypeptide is covalently linked to a carrier molecule.

8. Immunogenic composition according to any one of claims 1 to 7, which is in the form of a live attenuated vaccine comprising a recombinant live organism, of viral or bacterial origin, the said live organism expressing one or more polypeptide(s) as defined in any one of claim 1 to 7.

9. Immunogenic composition or polypeptide according to any one of claims 1 to 8, for use as a medicament.

10. Immunogenic composition or polypeptide according to any one of claims 1 to 8, for use as an agent for desensitization of individual with an IgE-dependent gluten allergy.

11. Immunogenic composition or polypeptide for its use according to claim 10, wherein the said IgE-dependent gluten allergy is selected in the group of durum wheat allergy, bread wheat allergy, spelt allergy, kamut allergy.

12. Immunogenic composition or polypeptide for its use according to claim 10 or 11, wherein the said IgE-dependent gluten allergy is a cross-allergy between α-gliadin allergy and γ-gliadin allergy.
